(19) Europäisches Patentamt
European Patent Office
Office européen des brevets

(11) EP 0 404 283 B1

(12) **EUROPÄISCHE PATENTSCHRIFT**

(45) Veröffentlichungstag und Bekanntmachung des Hinweises auf die Patenterteilung:
**08.05.1996 Patentblatt 1996/19**

(51) Int. Cl.$^6$: **C07J 43/00**, C07J 1/00, C07J 41/00, C07J 17/00, C07J 21/00, A61K 31/56

(21) Anmeldenummer: **90250142.8**

(22) Anmeldetag: **01.06.1990**

(54) **11-Beta-aryl-4-estrene, Verfahren zu ihrer Herstellung sowie deren Verwendung als Arzneimittel**

11-Beta-aryl-4-estrene, method for its production and its use as a medicine

11-Bêta-aryl-4-estrène, son procédé de préparation et son application comme médicament

(84) Benannte Vertragsstaaten:
**AT BE CH DE DK ES FR GB GR IT LI LU NL SE**

(30) Priorität: **23.06.1989 DE 3921059**

(43) Veröffentlichungstag der Anmeldung:
**27.12.1990 Patentblatt 1990/52**

(73) Patentinhaber: **SCHERING AKTIENGESELLSCHAFT**
**D-13342 Berlin (DE)**

(72) Erfinder:
- **Cleve, Arwed, Dr.**
**D-1000 Berlin 42 (DE)**
- **Scheidges, Cornelius, Dr.**
**D-1000 Berlin 33 (DE)**
- **Neef, Günter, Dr.**
**D-1000 Berlin 15 (DE)**
- **Ottow, Eckhard, Dr.**
**D-1000 Berlin 45 (DE)**
- **Elger, Walter, Dr.**
**D-1000 Berlin 33 (DE)**
- **Beier, Sybille, Dr.**
**D-1000 Berlin 31 (DE)**

(56) Entgegenhaltungen:
**EP-A- 0 192 598**
**EP-A- 0 196 707**
**EP-A- 0 277 089**
**EP-A- 0 308 345**
**WO-A-87/05908**
**WO-A-88/01868**
**AU-A- 59 584**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 404 283 B1

## Beschreibung

Die vorliegende Erfindung betrifft 11β-Aryl-4-estrene der allgemeinen Formel I

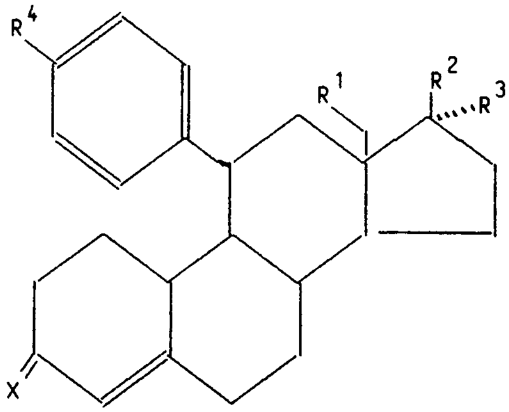

(I),

worin

X für ein Sauerstoffatom, die Hydroxyiminogruppierung >N~OH oder zwei Wasserstoffatome,

$R^1$ für ein Wasserstoffatom oder eine Methylgruppe,

$R^2$ für eine Hydroxygruppe, eine $C_1$-$C_{10}$-Alkoxy- oder $C_1$-$C_{10}$-Acyloxygruppe,

$R^3$ für ein Wasserstoffatom, die Gruppierung $-(CH_2)_nCH_2Z$, wobei n 0, 1, 2, 3, 4 oder 5 ist, Z ein Wasserstoffatom, die Cyanogruppe oder den Rest $-OR^5$ mit $R^5$=H, $C_1$-$C_{10}$-Alkyl oder $C_1$-$C_{10}$-Acyl bedeuten, die Gruppierung $-(CH_2)_mC{\equiv}C$-Y, wobei m 0, 1 oder 2 und Y ein Wasserstoff-, Fluor-, Chlor-Brom-oder Jod-Atom, einen $C_1$-$C_{10}$-Hydroxyalkyl-, $C_1$-$C_{10}$-Alkoxyalkyl-, $C_1$-$C_{10}$-Acyloxyalkylrest bedeuten, die Gruppierung $-(CH_2)_p$-CH=CH-$(CH_2)_kCH_2R^6$, wobei p 0 oder 1 und k 0, 1 oder 2 und $R^6$ ein Wasserstoffatom, eine Hydroxygruppe, einen $C_1$-$C_4$-Alkoxy- oder $C_1$-$C_4$-Acyloxyrest bedeuten,
oder aber $R^2$ und $R^3$ gemeinsam für einen Rest der Formel

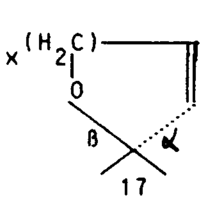

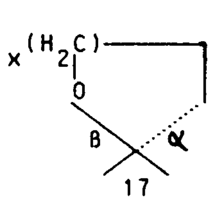

oder

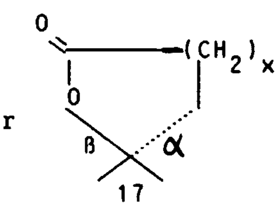

wobei x = 1 oder 2 ist

$R^4$ für ein Wasserstoffatom, eine Cyanogruppe, ein Chlor-, Fluor-, Brom-, Jodatom, für eine Trialkylsilyl-, Trialkylstannylgruppe, für einen geradkettigen oder verzweigten, gesättigten oder ungesättigten $C_1$-$C_8$-Alkyl-, -Acyl- oder Alkoxyalkylrest, für
eine Aminogruppe

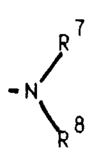

,

in welcher $R^7$ und $R^8$ unabhängig voneinander ein Wasserstoffatom oder eine $C_1$-$C_4$-Alkylgruppe bedeuten, oder für ein entsprechendes Aminoxid

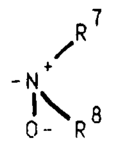,

oder für die Gruppierungen $-OR^9$ oder $-S(O)_iR^9$ mit i = 0, 1 oder 2, in welchen $R^9$ ein Wasserstoffatom, eine Methyl-, Ethyl-, Propyl-, Isopropyl-, Methoxyphenyl-, Allyl-oder eine 2-Dimethylaminoethylgruppe bedeuten, oder für einen Heteroarylrest der Formel Iα

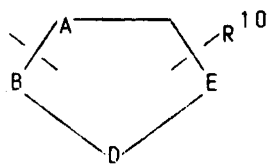 (Iα),

in welchem A ein Stickstoff-, Sauerstoff- oder Schwefelatom, -B-D-E- die Elementenfolge -C-C-C-, -N-C-C- oder -C-N-C- und $R^{10}$ ein Wasserstoffatom, eine Cyanogruppe, ein Chlor-, Fluor-, Brom-, Jodatom, eine Trialkylsilyl-, Trialkylstannylgruppe, einen geradkettigen oder verzweigten, gesättigten oder ungesättigten $C_1$-$C_8$-Alkyl-, -Acyl-oder Alkoxyalkylrest, für eine Aminogruppe

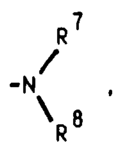,

in welcher $R^7$ und $R^8$ unabhängig voneinander ein Wasserstoffatom oder eine $C_1$-$C_4$-Alkylgruppe bedeuten, oder ein entsprechendes Aminoxid

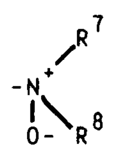,

oder die Gruppierung $-OR^9$ oder $-S(O)_iR^9$ mit i = 0, 1 oder 2, in welchen $R^9$ ein Wasserstoffatom, eine Methyl-, Ethyl-, Propyl-, Isopropyl-, Methoxyphenyl-, Allyl-oder eine 2-Dimethylaminoethylgruppe bedeuten,
symbolisieren,
oder für einen Heteroarylrest der Formel Iβ

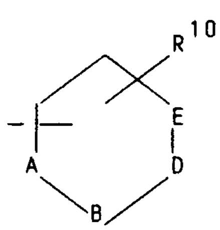 (IB)

in welchem A ein Stickstoffatom und -B-D-E- die Elementenfolge -C-C-C-, -N-C-C-, -C-N-C- oder -C-C-N- bedeuten unbd $R^{10}$ die bereits angegebene Bedeutung hat,
oder für einen Phenylrest der Formel Iγ

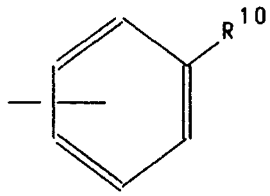

(Iγ),

worin $R^{10}$ die bereits angegebene Bedeutung hat,
stehen,
sowie deren pharmakologisch verträgliche Additionssalze mit Säuren, Verfahren zu ihrer Herstellung, diese Verbindungen enthaltende pharmazeutische Präparate, ihre Verwendung zur Herstellung von Arzneimitteln sowie die dazu benötigten neuen Zwischenprodukte.

Die Erfindung betrifft insbesondere Verbindungen, in denen X für ein Sauerstoffatom steht.

Die in $R^2$, $R^3$, $R^5$ und Y der allgemeinen Formel I enthaltenen Alkoxy-, Acyloxy-, Alkyl-, Acyl- sowie Hydroxyalkylgruppen sollen jeweils 1 bis 10 und die Alkoxyalkyl- oder Acyloxyalkylgruppen in Y 2 bis 10 Kohlenstoffatome enthalten. Dabei sind als bevorzugte Gruppen von den Alkoxygruppen die Methoxy-, Ethoxy-, Propoxy- und Isopropoxygruppe zu nennen, von den Acyl(oxy)-gruppen kommt der Formyl(oxy)-, Acetyl(oxy)- und Propionyl(oxy)-gruppe besondere Bedeutung zu.

Bei den Alkylgruppen sind vor allem die Methyl-, Ethyl-, Propyl-, Isopropyl-sowie tert.-Butylgruppe zu nennen und von den Hydroxyalkylgruppen sind die entsprechenden, in beliebiger Stellung mit einer Hydroxygruppe substituierten Reste bevorzugt.

Für n kommt insbesondere 0, 1, 2 und 3 infrage; wenn Z=CN, ist eine Cyanomethyl gruppe (n=0) besonders bevorzugt. Außer den bereits genannten Gruppen kann Y vorzugsweise auch ein Wasserstoff-, Chlor- oder Brom-Atom sein.

Von den Alkenylresten in $R^3$ sind die Propenyl- und Butenylgruppen, die in der E- oder Z-Konfiguration vorliegen können, bevorzugt, das heißt, wenn $R^3$ für $-(CH_2)_p-CH=CH-(CH_2)_k-CH_2-R^6$ steht, dann soll k vorzugsweise 0 oder 1 und p=0 sein.
Unter den für $R^6$ genannten Alkoxy- oder Acyloxygruppen, die sowohl geradkettig als auch verzweigt sein können, sind die Methoxy-, Ethoxy-, Propoxy-, Isopropoxy-bzw. die Formyloxy-, Acetyloxy- und Propionyloxy-gruppe besonders bevorzugt.

Von den $C_1$-$C_8$-Alkyl- und Alkoxyalkylresten, die für $R^4$ stehen können, sind dies vor allem der Methyl-, Ethyl-, Propyl-, Isopropyl-, Cyclopentyl- und Cyclohexylrest bzw. die Alkoxymethyl- bzw. 1- oder 2-Alkoxyethylgruppen mit den genannten Alkylresten; von den $C_1$-$C_8$-Acylresten für $R^4$ ist insbesondere an den Acetyl-, Propionyl- und Isobutylrylrest gedacht.

Steht $R^4$ für die Aminogruppe

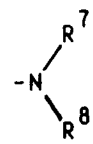

,

bedeuten $R^7$ und $R^8$ vorzugsweise jeweils einen Methylrest, doch auch dem Ethylrest kommt besondere Bedeutung zu, wobei dann entweder beide Reste am Stickstoffatom für einen Ethylrest oder einer für einen Methyl- und einer für einen Ethylrest stehen.
Für den Substituenten $R^9$ sind die Methyl-, Ethyl- und 2-(Dimethylamino)ethylgruppe besonders hervorzuheben.

Von dem gemäß Formel Iα möglichen Heteroarylresten sind der 3-Thienyl-, 3-Furyl- und 3-Pyrrolylrest bevorzugt mit $R^{10}$ in der Bedeutung einer Cyano-, Methoxy- oder Dimethylaminogruppe.

Als Heteroarylreste der Formel Iβ kommen erfindungsgemäß insbesondere der 3-oder 4-Pyridyl-, der 5-Pyrimidinyl-, 4-Pyridazinyl- oder Pyrazinylrest infrage. Der Phenylrest der Formel Iγ weist als Substituenten $R^{10}$ insbesondere die Cyano-, Methoxy- oder Dimethylaminogruppe auf, wobei sich wiederum diese Substituenten bevorzugt in der p-Position des Phenylringes befinden.

Nachstehend genannte Verbindungen sind erfindungsgemäß besonders bevorzugt:
11β-[4-(Dimethylamino)phenyl]-17β-hydroxy-17α-(1-propinyl)-4-estren-3-on;
11β-(4-Acetylphenyl)-17β-hydroxy-4-estren-3-on;
11β-(4-Acetylphenyl)-17β-hydroxy-17α-(1-propinyl)-4-estren-3-on;
11β-(4-Acetylphenyl)-17β-hydroxy-17α-(3-hydroxy-1Z-propenyl)-4-estren-3-on;

11β-[4-(Dimethylamino)phenyl]-17β-hydroxy-17α-(3-hydroxy-1Z-propenyl)-4-estren-3-on;
11β-[4-(3-Furyl)phenyl]-17β-hydroxy-17α-(1-propinyl)-4-estren-3-on;
11β-[4-(3-Furyl)phenyl]-17β-hydroxy-17α-(3-hydroxy-1Z-propenyl)-4-estren-3-on;
11β-[4-(5-Pyrimidinyl)phenyl]-17β-hydroxy-17α-(1-propinyl)-4-estren-3-on;
11β-[4-(5-Pyrimidinyl)phenyl]-17β-hydroxy-17α-(3-hydroxy-1Z-propenyl)-4-estren-3-on;
11β-[4-(3-Pyridyl)phenyl]-17β-hydroxy-17α-(1-propinyl)-4-estren-3-on;
11β-[4-(3-Pyridyl)phenyl]-17β-hydroxy-17α-(3-hydroxy-1Z-propenyl)-4-estren-3-on
11β-[4-(4-Cyanophenyl)phenyl]-17β-hydroxy-17α-(1-propinyl)-4-estren-3-on;
11β-[4-(4-Cyanophenyl)phenyl]-17β-hydroxy-17α-(3-hydroxy-1Z-propenyl)-4-estren-3-on;
11β-(4-Vinylphenyl)-17β-hydroxy-17α-(3-hydroxy-1Z-propenyl)-4-estren-3-on;
11β-(4-Vinylphenyl)-17β-hydroxy-17α-(1-propinyl)-4-estren-3-on;
11β-[4-(1-Hydroxyethyl)phenyl]-17β-hydroxy-17α-(3-hydroxy-1Z-propenyl)-4-estren-3-on;
11β-[4-(1-Hydroxyethyl)phenyl]-17β-hydroxy-17α-(1-propinyl)-4-estren-3-on;
11β-[4-(Dimethylamino)phenyl]-17β-hydroxy-17α-methoxymethyl)-4-estren-3-on;
11β-[4-(Dimethylamino)phenyl]-17β-hydroxy-17α-cyanomethyl)-4-estren-3-on;
(11β,17β)-4′,5′-Dihydro-11-[4-(dimethylamino)phenyl]spiro[estr-4-en-17,2′(3′H)-furan]-3-on;
(11β,17β)-3′,4′-Dihydro-11-[4-(dimethylamino)phenyl]spiro(estr-4-en-17,2′(5′H)-furan]-3,5-dion;
(11β,17β)-11-[4-(Dimethylamino)phenyl]spiro[estr-4-en-17,2′(5′H)-furan]-3-on;
11β-[4-(Dimethylamino)phenyl]-17α-(1-propinyl)-4-estren-17β-ol
17β-Hydroxy-3-oxo-11β-[4-(3-pyridinyl)phenyl]-4-estren-17α-acetonitril
(*E*)-17β-Hydroxy-3-(hydroxyimino)-11β-[4-(3-pyridinyl)phenyl]-4-estren-17α-acetonitril
(*Z*)-17β-Hydroxy-3-(hydroxyimino)-11β-[4-(3-pyridinyl)phenyl]-4-estren-17α-acetonitril
17β-Hydroxy-17α-(2-propenyl)-11β-[4-(3-pyridinyl)phenyl]-4-estren-3-on
17β-Hydroxy-17α-(methoxymethyl)-11β-[4-(3-pyridinyl)phenyl]-4-estren-3-on
11β-(4-Ethylphenyl)-17β-hydroxy-17α-(1-propinyl)-4-estren-3-on
(*Z*)-11β-(4-Ethylphenyl)-17β-hydroxy-17α-(3-hydroxy-1-propenyl)-4-estren-3-on
(*Z*)-11β-[4-(2-Furanyl)phenyl]-17β-hydroxy-17α-(3-hydroxy-1-propenyl)-4-estren-3-on
11β-(4-Ethenylphenyl)-17β-hydroxy-17α-methyl-4-estren-3-on
(*Z*)-17β-Hydroxy-17α-(3-hydroxy-1-propenyl)-11β-(4-methylphenyl)-4-estren-3-on
(11β,17β)-11-[4-(5-Pyrimidinyl)phenyl]spiro[estr-4-en-17,2′(3′H)-furan]-3-on.

Bei den Verbindungen der allgemeinen Formel I handelt es sich um kompetitive Antagonisten des Progesterons (Antigestagene). Alle bis vor kurzem bekannt gewordenen steroidalen Antigestagene weisen neben einem $\Delta^4\Delta^9$-3-Oxo-chromophor einen vorzugsweise substituierten 11β-Phenylrest auf (A. Belanger, D. Philibert und G. Teutsch, Steroids 37,2742 (1981); D. Philibert, T. Ojasoo und J.P. Raynand, Endocrinology 10,1850 (1977), EP-A 057 115; G. Teutsch, T. Ojasoo und J.P. Raynand, J. Steroid Biochem, 31, 549 (1988)).

Neuerdings sind auch Antigestagene steroidalen Ursprungs gefunden worden, in denen anstelle der 9,10-Doppelbindung eine Methylenbrücke zwischen dem 9-C-Atom und einem der ortho-C-Atome des 11β-Arylringes getreten ist (EP-A 0283428).

Offensichtlich bewirkt die Einführung des 11β-Arylrestes den Übergang von gestagener zu antigestagener Wirksamkeit. Allerdings ist es bisher nicht gelungen, ein dem Progesteron am nächsten kommendes Antigestagen, sozusagen "das Antiprogesteron", welches keine 9,10-Doppelbindung sondern neben einem 11β-Arylrest einen "freien" 10β-Substituenten, z.B. ein Wasserstoffatom aufweisen würde herzustellen. Versuche, 11β-[4-(Substituent)-aryl]-17β-hydroxy-5(10)-estren-3-on durch kurzzeitige Behandlung mit verdünnten Mineralsäuren in die entsprechende Verbindung mit einer 4(5)-Doppelbindung zu isomerisieren, Bedingungen, unter denen in der 11-unsubstituierten Reihe eine Doppelbindungsverschiebung von der 5(10)- in die 4(5)-Stellung ohne weiteres erfolgt, schlugen fehl (G. Neef, G. Sauer und R. Wiechert, Tet. Let., 24, 5205 (1983)).

Es wurden nunmehr jedoch Bedingungen gebunden, unter denen es überraschenderweise gelingt, eine Verschiebung der 5(10)-Doppelbindung in die 4(5)-Position zu bewerkstelligen.

Behandlung von Verbindungen der allgemeinen Formel II

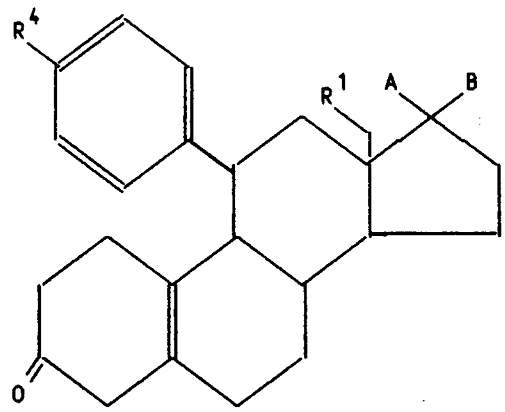

(II),

worin
$R^1$ und $R^4$ die in Formel I angegebene Bedeutung haben,
A für eine β-Hydroxygruppe oder den Rest $R^2$ und
B für ein α-Wasserstoffatom, einen α-ständigen Rest $R^3$ oder
A und B gemeinsam für ein Ketosauerstoffatom stehen,
mit Säure in einem inerten Lösungsmittel unter Erhitzen führt zu Verbindungen der allgemeinen Formel Ia

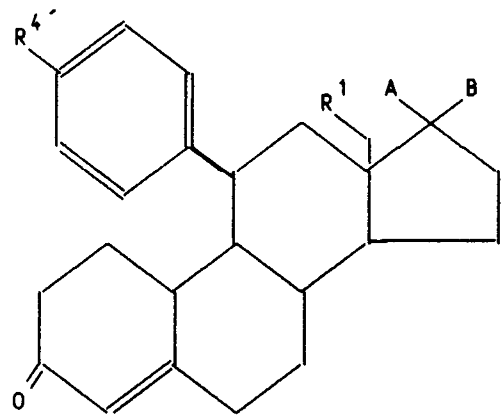

(Ia),

worin $R^1$, A und B die in Formel II angegebene Bedeutung haben und $R^{4'}$ dieselbe Bedeutung wie $R^4$ in Formel I hat, mit der Maßgabe, daß $R^4$ unter den genannten drastischen Reaktionsbedingungen stabil ist.

Vorzugsweise wird zur Isomerisierung auf eine Temperatur zwischen 80 und 120°C erhitzt, und zwar in einem inerten Lösungsmittel wie etwa Toluol.
Die Reaktionsdauer beträgt mindestens 45 Minuten, kann aber erforderlichenfalls 24 Stunden oder mehr betragen.
Als Säuren eignen sich sowohl Mineral- als auch organische Säuren; von letzteren ist p-Toluolsulfonsäure bevorzugt.

Bei den Verbindungen der allgemeinen Formel Ia kann es sich bereits um eine Endverbindung der allgemeinen Formel I handeln, wenn die Substituenten $R^4$, A und B in der Ausgangsverbindung der allgemeinen Formel II solche Substituenten sind, die den zur Isomerisierung benötigten drastischen Reaktionsbedingungen standhalten. Insbesondere freie Hydroxygruppen an einem tertiären Kohlenstoffatom eliminieren unter diesen Reaktionsbedingungen.

Es kann aber in jedem Fall sinnvoll sein, erst nach der Isomerisierung die Substituenten $R^2$ und $R^3$ am C-17-Atom einzuführen bzw. $R^4$ in der 4-Stellung des 11β-Phenylrestes aufzubauen.
Je nach den in der Verbindung der allgemeinen Formel I letztendlich gewünschten Substituenten $R^2$, $R^3$ und $R^4$ wird nach der Isomerisierung gegebenenfalls entweder

a) in der Verbindung der allgemeinen Formel Ia, wenn darin A für eine β-Hydroxygruppe und B für ein α-Wasserstoffatom stehen, gewünschtenfalls die 17-Hydroxy- zur 17-Ketogruppe oxidiert und
b) die 3-Ketofunktion in ein Dithioketal überführt, wobei auch alle anderen, gegebenenfalls vorhandenen Ketogruppen ketalisiert werden oder aber zuerst b) und dann a) ausführt und danach

c) für den Fall, daß $R^{4'}$ in der 3-thioketalisierten Verbindung für eine Methoxy-oder eine Hydroxygruppe steht und $R^4$ in der letztlich gewünschten Verbindung der allgemeinen Formel I nicht für eine Methoxy- oder Hydroxygruppe stehen soll,

die Hydroxyverbindung, gegebenenfalls nach Spaltung der Methoxyverbindung, in eine entsprechende Perfluoralkylsulfonsäureverbindung, in welcher -alkyl-für einen $C_1$-$C_4$Alkylrest, steht, überführt und aus dieser entweder direkt durch Umsetzung mit einer entsprechend substituierten Zinn(trialkyl)verbindung $R^{4''}$—Sn(Alkyl)$_3$ oder mit einer entsprechend substituierten Borverbindung $R^{4''}$—$BL_2$ (L=Hydroxy oder Alkyl), in welchen $R^{4''}$ mit $R^4$ der allgemeinen Formel I identisch ist oder einen tautomeren Vorläufer von $R^4$ darstellt und Alkyl einen $C_1$-$C_4$-Alkylrest bedeutet oder indirekt über eine in 4-Stellung des 11β-Phenylrestes mit einem Zinn(trialkyl)-rest (alkyl = $C_1$-$C_4$) substituierten Verbindung die durch Umsetzung der Perfluoralkylsulfonatverbindung mit $Sn_2alkyl_6$ erhalten wurde, und Weiterbehandlung der 11β-(4-Trialkylstannyl)-phenyl-Verbindung mit einer Verbindung $R^{4''}$-Y, worin $R^{4''}$ mit $R^4$ der allgemeinen Formel I identisch ist oder einen tautomeren Vorläufer von $R^4$ darstellt und Y eine Abgangsgruppe, vorzugsweise ein Halogenatom und insbesondere ein Bromatom bedeutet, in Gegenwart eines Übergangsmetall-Katalysators eine Verbindung der allgemeinen Formel III

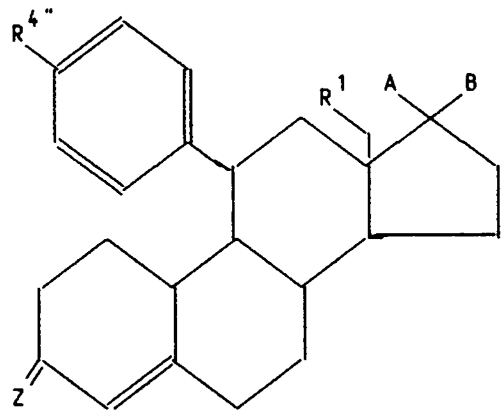

(III),

worin Z eine in Form eines Dithioketals geschützte Ketogruppe bedeutet, hergestellt und

d) dann, falls $R^2$ und $R^3$ in der letztlich gewünschten Verbindung der allgemeinen Formel I nicht für eine Hydroxygruppe bzw. ein Wasserstoffatom oder aber $R^2$ und $R^3$ gemeinsam nicht für ein Ketosauerstoffatom stehen sollen, am C-17-Atom des Steroidgerüstes die gewünschten Substituenten $R^2$ und $R^3$ nach an sich bekannten Methoden eingeführt

oder aber zuerst d) und dann c) ausgeführt wird,

Schutzgruppen abgespalten, gewünschtenfalls freie Hydroxygruppen alkyliert bzw. acyliert werden und gewünschtenfalls mit Hydroxylaminhydrochlorid die 3-Ketogruppe in eine 3-Hydroxyiminogruppierung >N~OH oder die 3-Ketogruppe in die Dihydroverbindung überführt sowie gegebenenfalls ein pharmazeutisch verträgliches Additionssalz mit einer Säure hergestellt wird.

Die Durchführung der Verfahrensschritte a), b), c) und d) erfolgt nach an sich bekannten Methoden.

Die Oxidation a) der Hydroxy- zur Ketogruppe kann beispielsweise nach Oppenauer oder mit Chromsäurereagenzien (Jones'-Reagenz) oder Chromsäure-Pyridin vorgenommen werden.

Als Schutzgruppe für die 3-Ketofunktion dient vorzugsweise die Ethan-1,2-diyl-bis(thio)-Gruppe, die durch Umsetzung der 3-Ketoverbindung mit Ethan-1,2-dithiol in Gegenwart von z.B. p-Toluolsulfonsäure am Steroid-3-C-Atom eingeführt werden kann.

Wahlweise kann zuerst die Oxidation und dann die Schutzgruppeneinführung oder aber zuerst die Schutzgruppeneinführung und dann die Oxidtion durchgeführt werden.

Der Reaktionsschritt c) dient dem Aufbau des Substituenten $R^4$ bzw. $R^{4'}$ in p-Stellung am 11β-Phenylring. Diese Vorgehensweise ist dann erforderlich, wenn $R^4$ einen solchen Substituenten darstellt, der den drastischen Isomerisierungsbedingungen nicht standhält, beispielsweise ein Allyl- oder Vinylrest.

Als Ausgangspunkt für diesen Aufbau dient die 11β-(4-Hydroxyphenyl)-verbindung, die aus der entsprechenden Methoxyverbindung durch Etherspaltung, beispielsweise mit Natriummethanthiolat in einem Lösungsmittel wie Dimethylformamid, erhältlich ist.

Durch Umsetzung der Hydroxyverbindung mit einem Perfluor-($C_1$-$C_4$)-alkylsulfonsäureanhydrid oder -halogenid in Gegenwart einer Base wie Pyridin oder 4-(Dimethylamino)-pyridin gelangt man zur entsprechenden 11β-[4-(perfluoralkylsulfonyloxy)phenyl]-Verbindung (P.J. Stang, M. Hanack und L.R. Subramanian, Synthesis 85, (1982)).

Bei der sich anschließenden Kupplung der 11β-Arylverbindung mit $R^{4''}$ -Sn(Alkyl)$_3$ oder $R^{4''}$-$BL_2$ wird entweder so vorgegangen, daß in einer übergangsmetallkatalysierten Reaktion (vorzugsweise $Pd^0$) die Perfluoralkylsulfonatabgangsgruppe unter im wesentlichen fast gleichzeitiger Substitution durch den gewünschten Substituenten oder dessen Vorstufe verdrängt wird (Arylkupplungen mit Zinnverbindungen: J.E. McMurry and S. Mohanraj, Tetrahedron Letters, 24, No. 27, S. 2723-2726, 1983; X. Lu und J. Zhu, Communications, S. 726-727, 1987; Q. - Y. Chen und Z.-Y. Yang, Tetrahedron Letters 27, No. 10, S. 1171-1174, 1986; S. Cacchi, P.G. Ciattini, E. Morera und G. Ortar, Tetrahedron Letters, 27, No. 33, S. 3931-3934, 1986; A.M. Echavarren und J.K. Stille, J. Am. Chem. Soc. 1987, 109, S. 5478-5486; mit Borverbindungen: Synthesis 936 (1984), Chem.Pharm.Bull. 33,4755-4763 (1985); J.Org.Chem.49, 5237-5243 (1984); Bull.Chem.Soc.Jpn.61, 3008-3010ß (1988) oder es wird aus der Perfluoralkylsulfonat-Verbindung intermediär und übergangsmetallkatalysiert eine entsprechende Tri-organylstannyl-, vorzugsweise Tri-n-alkylstannyl-Verbindung hergestellt [J.K. Stille, Angew. Chem. 98 (1986), S. 504-519]. Diese wird anschließend in einer Eintopf-Reaktion mit einem halogen-, vorzugsweise brom-oder jodsubstituierten carbocyclischen oder heterocyclischen Aromaten [Y. Yamamoto, Y. Azuma, H. Mitoh, Communications, S. 564-565, 1986; T. J. Bailey, Tetrahedron Letters, 27, No. 37, S. 4407-4410, 1986], der gegebenenfalls noch weitere Substituenten tragen kann, umgesetzt; der 11β-Phenylrest weist darin dann die gewünschte bzw. einen Vorläufer der gewünschten Substitution auf.
Zahlreiche derartige Umsetzungen mit Steroiden, in denen eine Trifluormethansulfonat-Gruppe sich in 4-Stellung eines 11β-Phenylringes befindet, sind in der EP-A-0283428 beschrieben.
Freie Hydroxygruppen können in an sich bekannter Weise alkyliert oder acyliert werden.
Dialkylamine können durch geeignete Oxidationsmittel (z.B. Wasserstoffperoxid bzw. Persäuren) in die gewünschten N-Oxide [siehe z.B. Kontakte (Darmstadt) 1986,3,S.12] überführt werden.

Verbindungen mit einem Dialkylamin-Substituenten am 11β-Phenylring können durch Reaktion mit Bromcyan in aprotischen Lösungsmitteln wie zum Beispiel Dioxan, Benzol oder Toluol bei erhöhter Temperatur (Amin-Abbau nach Braun) analog den zum Beispiel in Org. Reactions 7, 198 (1953), K.W. Bentley, Techniques of Organic Chemistry 11, 773 (1963) und Houben-Weyl, 5/4, 151 (1960) angegebenen Vorschriften in guter Ausbeute in die entsprechenden (N-Cyan-N-alkylaminoaryl)-derivate überführt werden.

Diese werden je nach der letztlich gewünschten Bedeutung von

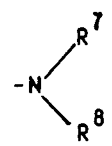

im Endprodukt in an sich bekannter Weise zu den entsprechenden Dialkylamin-Verbindungen (zum Beispiel mit Diisobutylaluminiumhydrid in Toluol zu den N-Formyl-N-alkylaminophenyl-Zwischenprodukten und anschließend mit Lithiumaluminiumhydrid) bzw. N-H-N-alkyl-Verbindungen reduziert (zum Beispiel mit Lithiumaluminiumhydrid oder mit Lithium in flüssigem Ammoniak). Die letzteren werden anschließend gewünschtenfalls in literaturbekannter Weise acyliert und gegebenenfalls anschließend in bekannter Weise mit zum Beispiel Lithiumaluminiumhydrid zum neuen Dialkylaminderivat reduziert (s. DE 36 23 038).

Im Verfahrensschritt d) schließlich werden die letztlich am 17-C-Atom gewünschten Substituenten $R^2$ und $R^3$ eingeführt, falls es sich dabei nicht um eine schon von vornherein als $R^2$ enthaltene Methoxy- oder Hydroxygruppe bzw. ein Wasserstoffatom als $R^3$ oder um ein gemeinsam aus $R^2$ und $R^3$ gebildetes Ketosauerstoffatom handelt. Diese Einführung erfolgt analog literaturbekannten Verfahren (zum Beispiel J. Fried, J.A. Edwards, "Organic Reactions in Steroid Chemistry", Van Nostrand Reinhold Company, 1972, Vol. 1 und 2; "Terpenoids and Steroids", Specialist Periodical Report, The Chemical Society, London, Vol. 1- 2) durch nucleophile Addition an das C-17-Keton.

Die Einführung des Substituenten -C≡C-Y als $R^3$, wobei Y die oben angegebene Bedeutung hat, erfolgt mit Hilfe einer metallierten Verbindung der allgemeinen Formel MC≡C-Y', in der Y' eine Alkin- Schutzgruppe, wie zum Beispiel Trimethylsilyl oder tert.-Butyldimethylsilyl, ist.

Die metallorganische Verbindung kann auch in situ gebildet und mit dem 17-Keton zur Reaktion gebracht werden. So kann man zum Beispiel auf das 17-Keton in einem geeigneten Lösungsmittel Acetylen und ein Alkalimetall, insbesondere Kalium, Natrium oder Lithium, in Gegenwart eines Alkohols oder in Gegenwart von Ammoniak einwirken lassen. Das Alkalimetall kann auch in Form von zum Beispiel Methyl- oder Butyllithium zur Einwirkung kommen. Als Lösungsmittel sind insbesondere Dialkylether, Tetrahydrofuran, Dioxan, Benzol und Toluol geeignet.

Die Einführung von 3-Hydroxypropin, -propen bzw. -propan in 17-Stellung erfolgt durch Umsetzung des 17-Ketons mit dem Dianion des Propargylalkohols (3-Hydroxypropin), zum Beispiel dem in situ generierten Dikaliumsalz des Propargylalkohols, zum 17α-(3-Hydroxyprop-1-inyl)-17β-hydroxyderivat oder mit metallierten Derivaten des 3-Hydroxypropins, zum Beispiel mit 1-Lithium-3-(tetrahydropyran-2′-yloxy)-prop-1-in-1-id, zum 17-[3-(Tetrahydropyran-2′-yloxy)-prop-1-inyl]-17β-hydroxyderivat, die anschließend zu den 17-(3-Hydroxypropyl- bzw. Hydroxypropenyl)17β-hydroxy-Verbindungen hydriert werden können. Das gelingt zum Beispiel durch Hydrierung bei Raumtemperatur und Normaldruck in Lösungsmitteln wie Methanol, Ethanol, Propanol, Tetrahydrofuran (THF) oder Essigester unter Zusatz von Edelmetall-Katalysatoren wie Platin oder Palladium.

Die Einführung homologer Hydroxyalkin-, Hydroxyalken- und Hydroxyalkangruppen erfolgt in entsprechender Weise mit Homologen des Propargylalkohols.

Die Verbindung mit der Z-konfigurierten Doppelbindung in der Hydroxypropenylgruppe entsteht durch Hydrieren der acetylenischen Dreifachbindung mit einem desaktivierten Edelmetallkatalysator (J. Fried, J.A. Edwards: Organic Reactions in Steroid Chemistry, Van Nostrand Reinhold Company 1972, Seite 134; und H.O. House: Modern Synthetic Reactions 1972, Seite 19).Als desaktivierte Edelmetallkatalysatoren kommen beispielsweise 10% Palladium auf Bariumsulfat in Gegenwart eines Amins oder 5 % Palladium auf Calciumcarbonat unter Zusatz von Blei(II)-acetat infrage. Die Hydrierung wird nach der Aufnahme von einem Äquivalent Wasserstoff abgebrochen.

Die Verbindung mit der E-konfigurierten Doppelbindung in der Hydroxypropenylgruppe entsteht durch Reduktion der acetylenischen Dreifachbindung in an sich bekannter Weise. In der Literatur sind eine ganze Reihe von Methoden zur Umwandlung von Alkinen in trans-Olefine beschrieben, beispielsweise die Reduktion mit Natrium in flüssigem Ammoniak (J. Am. Chem. Soc. 63 (1941) 216), mit Natriumamid in flüssigem Ammoniak (J. Chem. Soc. 1955, 3558), mit Lithium in niedermolekularen Aminen (J. A. Chem. Soc. 77 (1955) 3378), mit Boranen (J. Am. Chem. Soc. 93 (1971) 3395 und 94 (1972) 6560), mit Diisobutylaluminiumhydrid und Methyl-Lithium (J. Am. Chem. Soc. 89 (1967) 5085) und insbesondere mit Lithiumaluminiumhydrid/Alkoholat (J. Am. Chem. Soc. 89 (1967) 4245). Eine weitere Möglichkeit ist die Reduktion der Dreifachbindung mit Chrom(II)-sulfat in Gegenwart von Wasser oder Dimethylformamid in schwach sauren Milieu (J. Am. Chem. Soc. 86 (1964) 4358) sowie allgemein die Reduktion durch einwirkung von Übergangsmetallverbindungen unter Wechsel der Oxydationsstufe.

Die Einführung der Hydroxyalkene kann auch direkt erfolgen durch Addition einer entsprechenden metallierten Hydroxyalkenylverbindung, wie zum Beispiel 1-Lithium-3-(tetrahydropyran-2'-yloxy)-prop-1(E)-en (J. Org. Chem. 40 2265) oder 1Lithium-3-(tetrahydropyran-2'-yloxy)-prop-1(Z)-en (Synthesis 1981, 999) Homologe können auf diese Art ebenfalls eingeführt werden.

Die Einführung von 3-Hydroxypropan in 17-Stellung kann ebenfalls direkt durch Umsetzung des 17-Ketons mit metallierten Derivaten von 3-Halogen-propanolen - wobei die Hydroxygruppe im Metallierungsschritt als Alkoholat (Tetrahedron Letters 1978, 3013) oder als geschützte Funktion (J. Org. Chem. 37, 1947) vorliegt - zu der 17-(3-Hydroxypropyl)-17β-hydroxy-verbindung bzw. zu der an der terminalen Hydroxygruppe geschützten Verbindung erfolgen. Als Schutzgruppen kommen zum Beispiel die Ethoxyethyl-, Tetrahydropyranyl- und Methoxymethyl-Gruppen in Frage.
Werden Endprodukte der Formel I gewünscht mit $R^2/R^3$ in der Bedeutung von

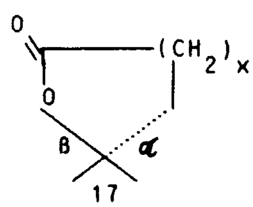

x = 1 oder 2 so wird die 17-(3-Hydroxypropyl)- bzw. 17-(4-Hydroxybutyl)-Verbindung in an sich bekannter Weise oxydiert, zum Beispiel mit Jones' Reagenz, Braunstein, Pyridiniumdichromat, Pyridiniumchlorochromat, Chromsäure-Pyridin oder dem Fetizon-Reagenz Silbercarbonat/Celite (Compt. rend. 267 [1968] 900).

Die Darstellung von Endprodukten der Formel I mit $R^2/R^3$ in der Bedeutung von

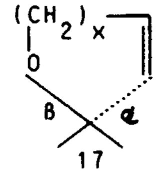

x = 1 oder 2 erfolgt durch Ringschlußreaktion des entsprechenden 17-(3-Hydroxyprop-1-(Z)-enyl-bzw. 17-(4-Hydroxybut-1-(Z)-enyl-17-β-hydroxy-Eduktes. Hydrierung des ungesättigten 5- oder 6-Ring-Spiroethers am Palladium/Aktivkohle-Kontakt führt zu den gesättigten Spiroethern.

Der Aufbau der 17-Cyanmethylseitenkette erfolgt in an sich bekannter Weise aus dem 17-Keton zum Beispiel über das 17-Spiroepoxid und Spaltung des Spiroepoxids mit HCN gemäß Z. Chem. 18 (1978) 259-260.

Auch die Einführung der 17-Hydroxyacetylseitenkette erfolgt nach an sich bekannten Methoden, beispielsweise nach den in J. Org. Chem. 47 (1982), 2993-2995, Chem.Ber. 113 (1984), 1184 bzw. US-Patent 4.600.538 beschriebenen Methoden.
Freie Hydroxygruppen können in an sich bekannter Weise alkyliert oder acyliert werden.

Gegebenenfalls kann auch zuerst die Einführung der Substituenten $R^2$ und $R^3$ wie unter d) beschrieben, vorgenommen werden und anschließend gemäß c) der Substituent $R^4$ aufgebaut werden, je nachdem, ob die Verfahrensbedingungen des zweiten Reaktionsschrittes die zuerst eingeführten aber aufgebauten Substituenten beeinträchtigen.

Noch vorhandene Schutzgruppen werden nach gängigen Methoden abgespalten.

Die erhaltenen Verbindungen der allgemeinen Formel I mit X in der Bedeutung eines Sauerstoffatoms können gewünschtenfalls durch Umsetzung mit Hydroxylaminhydrochlorid in Gegenwart von tertiären Aminen bei Temperaturen zwischen -20 und +40°C in die Oxime (Formel I mit X in der Bedeutung der Hydroxyiminogruppierung N OH, wobei die Hydroxygruppe syn- oder antiständig sein kann) überführt werden. Geeignete tertiäre Basen sind beispielsweise Trimethylamin, Triäthylamin, Pyridin, N,N-Dimethylaminopyridin, 1,5-Diazabicyclo[4.3.0]non-5en (DBN) und 1,8-Diazabicyclo[5.4.0]undec-7-en (DBU), wobei Pyridin bevorzugt ist.

Die Entfernung der 3-Oxogruppe zu einem Endprodukt der allgemeinen Formel I mit X in der Bedeutung von 2 Wasserstoffatomen kann z.B. nach der in der DE-A-2805490 angegebenen Vorschrift durch reduktive Spaltung des Thioketals erfolgen.

Die neuen Verbindungen der allgemeinen Formel I sowie deren Additionssalze mit pharmazeutisch verträglichen Säuren sind wertvolle Pharmaka. So verfügen sie über eine starke Affinität zum Gestagenrezeptor und besitzen überraschend starke antigestagene sowie antiglucocorticoide, antimineralcorticoide und antiandrogene Eigenschaften. Diese wichtigen biologischen Wirksamkeiten können für medizinische Zwecke genutzt werden.
Wirkstoffe dieser Art mit ausgeprägter antigestagener Aktivität sind zur Auslösung von Aborten geeignet, da sie das zur Aufrechterhaltung der Schwangerschaft erforderliche Progesteron vom Rezeptor verdrängen. Sie sind deshalb wertvoll und interessant im Hinblick auf ihre Verwendung zur postcoitalen Fertilitätskontrolle.
Die neuen Verbindungen können außerdem zur Behandlung der Endometriose dienen. Sie können auch gegen hormonelle Unregelmäßigkeiten, zur Menstruationsauslösung und zur Geburtseinleitung eingesetzt werden.
Außerdem können sie für die Behandlung von hormonabhängigen Carcinomen eingesetzt werden.

Die erfindungsgemäßen Verbindungen der allgemeinen Formel I sowie deren Additionssalze mit pharmazeutisch verträglichen Säuren weisen auch eine antiglucocorticoide Aktivität auf und können somit auch als Arzneimittel zur Therapie corticoid-induzierter Störungen (Glaukom) sowie zur Bekämpfung von Nebenwirkungen, die bei langfristiger Behandlung mit Glucocorticoiden auftreten (Cushing-Syndrom) eingesetzt werden. Sie ermöglichen daher auch die auf eine Supersekretion der Glucocorticoide zurückzuführenden Störungen, vor allem die Adipositas, Arteriosklerose, Hypertension, Osteoporose, Diabetes sowie die Insomnie zu bekämpfen.

Die erfindungsgemäßen Verbindungen der allgemeinen Formel I sowie deren Additionssalze mit pharmazeutisch verträglichen Säuren mit antiandrogener Aktivität können bei der Behandlung der Hypertrophie und des Prostatakarzinoms verwendet werden. Sie ermöglichen weiterhin eine spezifische Therapie von Androgenisierungserscheinungen bei Frauen: die pathologische Behaarung bei Hirsutismus, die androgenetische Alopezie sowie die gesteigerte Talgdrüsenfunktion bei Akne und Seborrhoe sind günstig beeinflußbar.

Die Erfindung betrifft somit auch Arzneimittel auf Basis der Verbindungen der allgemeinen Formel I sowie deren Additionssalze mit pharmazeutisch verträglichen Säuren, gegebenenfalls zusammen mit den üblichen Hilfs- und Trägerstoffen.

Die erfindungsgemäßen Verbindungen und deren Salze können nach an sich bekannten Methoden der Galenik zu pharmazeutischen Präparaten für die enterale, perkutane, parenterale oder lokale Applikation verarbeitet werden. Sie können in Form von Tabletten, Dragees, Gelkapseln, Granulaten, Suppositorien, Implantaten, injizierbaren sterilen wäßrigen oder öligen Lösungen, Suspensionen oder Emulsionen, Salben, Cremes und Gelen verabreicht werden.

Der oder die Wirkstoffe können dabei mit den in der Galenik üblichen Hilfsstoffen wie z.B. Gummiarabikum, Talk, Stärke, Mannit, Methylcellulose, Laktose, Tensiden wie Tweens$^{(R)}$ oder Myrj$^{(R)}$, Magnesiumstearat, wäßrigen oder nichtwäßrigen Trägern, Paraffinderivaten, Netz-, Dispergier-, Emulgier-, Konservierungsmitteln und Aromastoffen zur Geschmackskorrektur (z.B. ätherischen Ölen) gemischt werden.
Die Erfindung betrifft somit auch pharmazeutische Zusammensetzungen, die als Wirkstoff zumindest eine erfindungsgemäße Verbindung oder eines ihrer Additionssalze mit pharmazeutisch verträglichen Säuren enthalten.
Als Additionssalze der erfindungsgemäßen Produkte mit Säuren sind insbesondere die Hydrochloride und die Methansulfonate zu nennen.
Eine Dosiseinheit enthält etwa 1-100 mg Wirkstoff(e).
Die Dosierung der erfindungsgemäßen Verbindungen liegt beim Menschen bei etwa 1-1000 mg pro Tag.

Zur Kennzeichnung der antigestagenen Wirkung wurde die abortive Wirkung bestimmt.

Die Versuche wurden an weiblichen Ratten im Gewicht von ca. 200 g durchgeführt. Nach erfolgter Anpaarung wurde der Schwangerschaftsbeginn durch Nachweis von Spermien in Vaginalabstrichen gesichert. Der Tag des Spermiennachweises gilt als Tag 1 der Gravidität (= d1 p.c.).

Die Behandlung der Tiere mit der jeweils zu testenden Substanz bzw. dem Lösungsmittel erfolgte nach der Nidation der Blastocysten von d5 p.c. bis d7 p.c. An d9 p.c. wurden die Tiere getötet und die Uteri auf Implantate und Resorptionsstellen hin untersucht. Von allen Uteri wurden Fotos angefertigt. Das Fehlen von Implantaten, pathologische, hämorrhagische oder sonst abnorme Nidationsstellen, wurde als Abort gewertet.

Die Testsubstanzen wurden in einem Benzylbenzoat-Rizinusöl-Gemisch (Verhältnis 1 + 4) gelöst. Das Vehikelvolumen pro Einzeldosis betrug 0,2 ml. Die Behandlung erfolgte subcutan.

Die Überlegenheit der erfindungsgemäßen Verbindungen wird eindeutig durch Vergleich der abortiven Wirksamkeit der Verbindungen A bis E mit der in der europäischen Patentschrift 0 057 115 beschriebenen Verbindung F belegt; die Vergleichsverbindung F (= RU 486) unterscheidet sich von Verbindung A lediglich durch die zusätzliche 9(10)-Doppelbindung.

Als Antigestagene wurden untersucht:

A: 11β-[4-(Dimethylamino)phenyl]-17β-hydroxy-17α-(1-propinyl)-4-estren-3-on
B: (Z)-11β-[4-(Dimethylamino)phenyl]-17β-hydroxy-17α-(3-hydroxy-1-propenyl)-4-estren-3-on
C: (Z)-11β-(4-Acetylphenyl)-17β-hydroxy-17α-(3-hydroxy-1-propenyl)-4-estren-3-on
D: (Z)-17β-Hydroxy-17α-(3-hydroxy-1-propenyl)-11β-[4-(3-pyridinyl)phenyl]-4-estren-3-on
E: (Z)-4′-[17β-Hydroxy-17α-(3-hydroxy-1-propenyl)-3-oxo-4-estren-11β-yl][1,1′-biphenyl]-4-carbonitril
F: 11β-[4-(Dimethylamino)phenyl]-17β-hydroxy-17α-(1-propinyl)-4,9(10)estradien-3-on

Tabelle 1

| Abortivtest bei der graviden Ratte Behandlung von d5 p.c. bis d7 p.c., Autopsie an d9 p.c. | | | |
|---|---|---|---|
| Verbindung | Dosis mg/Tier/Tag s.c. | Abortrate n Abort / n Gesamt | % |
| A | 3.0 | 4/4 | (100) |
| | 1.0 | 4/4 | (100) |
| | 0.3 | 3/4 | ( 75) |
| B | 3,0 | 4/4 | (100) |
| | 1,0 | 4/4 | (100) |
| | 0,3 | 4/4 | (100) |
| C | 3,0 | 4/4 | (100) |
| | 1,0 | 4/4 | (100) |
| | 0,3 | 4/4 | (100) |
| | 0,1 | 4/4 | (100) |
| D | 3,0 | 4/4 | (100) |
| | 1,0 | 4/4 | (100) |
| | 0,3 | 4/4 | (100) |
| E | 3,0 | 4/4 | (100) |
| | 1,0 | 4/4 | (100) |
| | 0,3 | 4/4 | (100) |
| | 0,1 | 4/4 | (100) |
| F | 3.0 | 4/4 | (100) |
| | 1.0 | 2/4 | ( 50) |
| | 0,3 | 0/4 | ( 0) |
| Lösungsmittel als Kontrolle: | | | |
| 0.2 ml Benzylbenzoat + Rizinusöl (1+4) | - | 0/5 | ( 0) |
| n = 4 Ratten | | | |

Herstellung der benötigten Zwischenprodukte der allgemeinen Formel II

Die Herstellung der für alle Synthesen benötigten Ausgangsverbindung A, 3,3-[ ,2-Dimethyl-1,3-propandiylbis(oxy)]-5α,10α-epoxy-estr-9(11)-en-17β-ol, ist in EP-A-0110434 oder EP-A-0127864 beschrieben.

Für $R^4$= $-N(CH_3)_2$ ist die Darstellung von 11β-[4-(Dimethylamino)phenyl]-17β-hydroxy-17α-(1-propinyl)-5(10)-estren-3-on (1M) als einem Vertreter einer Verbindung der allgemeinen Formel II angegeben.

Durch Verwendung eines anderen nucleophilen Reagenzes als Propin im Syntheseschritt 1L, unter Bedingungen wie in der Beschreibung vorstehend angegeben, lassen sich auch die anderen erfindungsgemäß vorgesehen Reste $R^2$/$R^3$ einführen.

Im Beispiel 2 ist die Herstellung von 3,3-[2,2-Dimethyl-1,3-propandiylbis(oxy)] 10β,11β-(5-methoxy-o-phenylenthio)-estran-5α,17β-diol gezeigt.
Wie im Beispiel 1 beschrieben, gegebenenfalls unter Verwendung eines anderen nucleophilen Reagenzes als Propin bei der analogen Durchführung des Schrittes 1L, gelangt man zu Verbindungen der allgemeinen Formel II, worin $R^{4'}$ für eine Methoxygruppe steht.

Weitere Ausgangsprodukte der allgemeinen Formel II können wie im nachstehenden Reaktionsschema wiedergegeben hergestellt werden (die Bedeutungen der Substituenten $R^1$, $R^2$, $R^3$, $R^{4'}$ und $R^4$ sind identisch mit denen der allgemeinen Formeln):
Bei den Verbindungen D, E und L handelt es sich um Verbindungen der allgemeinen Formel II.
Der Reaktionsschritt F → I wird mit solchen Verbindungen F ausgeführt, in denen $R^{4'}$ für eine Hydroxygruppe steht. Diese wird gegebenenfalls durch Spaltung des entsprechenden Methylethers eingeführt.
Die Schritte F→ I sowie I → K werden analog wie die nach der Isomerisierung durchgeführte und beschriebene Triflatbildung mit nachfolgender Kupplung in 4-Stellung des 11β-Arylrestes durchgeführt.

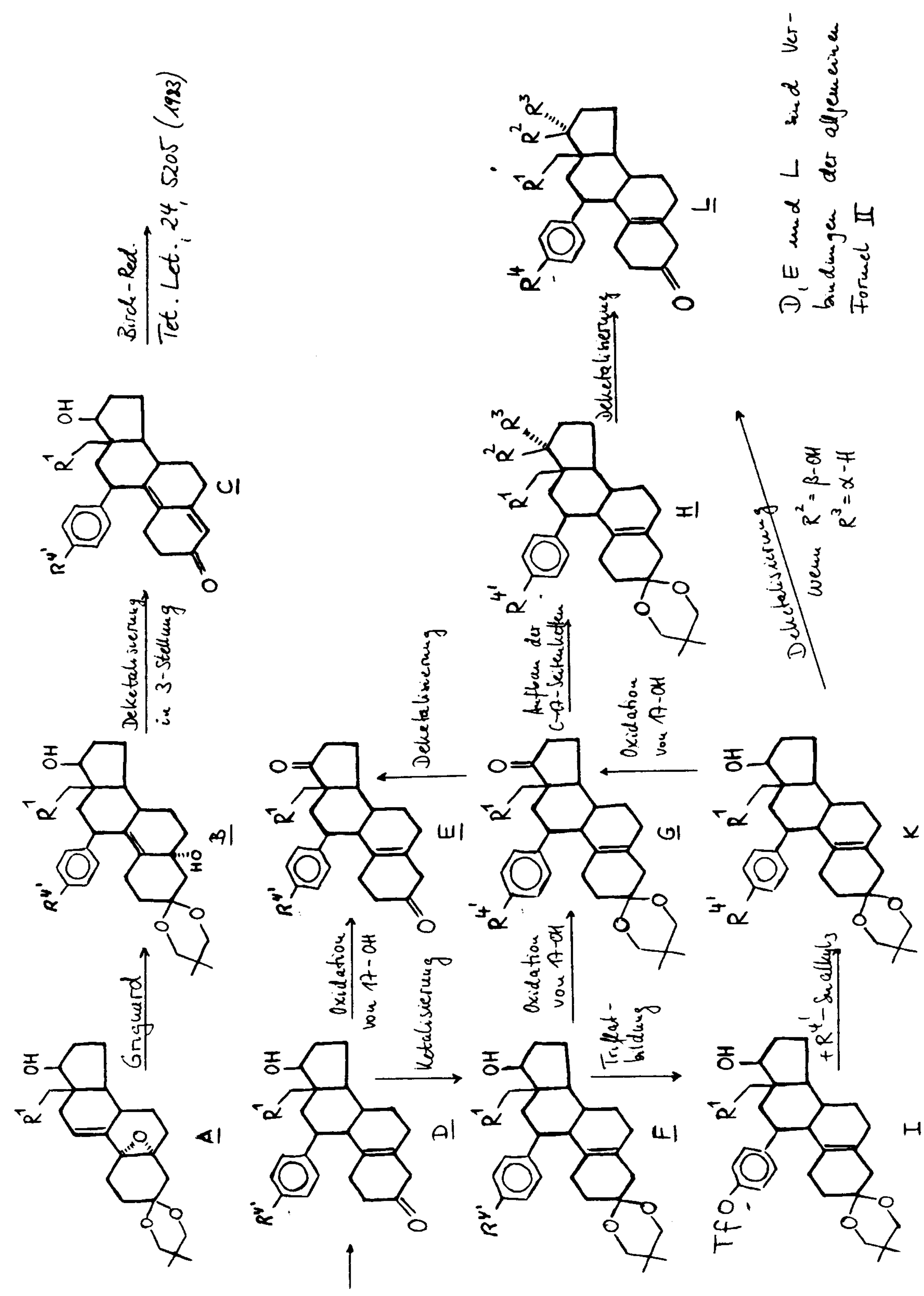


Die säulenchromatographischen Reinigungsschritte in den nachfolgenden Beispielen werden, insoweit nicht anders erwähnt, an Kieselgel mit Hexan/Essigester, gegebenenfalls mit einem Gemisch steigender Polarität, durchgeführt.

**BEISPIEL 1**

Darstellung von 11β-[4-(Dimethylamino)-phenyl]-17β-hydroxy-17α-(1-propinyl)-4-estren-3-on (1N)

A. 10β-[(3-Aminophenyl)thio]-3,3-[2,2-dimethyl-1,3-propandiylbis(oxy)]-9(11)-estr-en-5α,17β-diol (1A)

750 mg 3-Mercaptoanilin werden in 5 ml absolutem THF gelöst und bei - 40°C mit 3,73 ml einer 1,6 molaren n-Butyllithium-Lösung in Hexan versetzt. Es wird die Kühlung entfernt und 30 Minuten gerührt. Nun werden 500 mg 3,3-[2,2-Dimethyl-1,3-propandiylbis(oxy)]-5α,10α-epoxy-estr-9(11)-en-17β-ol, gelöst in 4 ml absolutem THF zugetropft, wobei die Temperatur bei 0°C gehalten wird. Anschließend wird 2 Stunden bei Raumtemperatur nachgerührt.
Die Reaktionsmischung wird mit 15 ml gesättigter $NH_4Cl$-Lösung versetzt und mit Essigester extrahiert, die organische Phase mit Wasser gewaschen und über $Na_2SO_4$ getrocknet, das Lösungsmittel im Vakuum abgezogen. Säulenchromatographie ergibt 484 mg 1A als Schaum.

B. 3,3-[2,2-Dimethyl-1,3-propandiylbis(oxy)]-10β-[(3-(formylamino)phenyl]thio)-9(11)-estr-en-5α,17β-diol (1B)

0,4 ml Acetanhydrid und 0,17 ml Ameisensäure werden zusammengegeben und bei Raumtemperatur unter Argon 15 min gerührt. Zu dieser Lösung fügt man 1,0 g 1A gelöst in 5 ml Pyridin p.a. und rührt bei Raumtemperatur unter Argon 2 Stunden nach.
Anschließend wird das Reaktionsgemisch in Essigester aufgenommen, mit gesättigter $NaHCO_3$-Lösung geschüttelt bis ein pH-Wert von ca. 8 erreicht ist, mit Wasser gewaschen, über $Na_2SO_4$ getrocknet und am Rotationsverdampfer eingeengt.
Ausbeute nach Säulenchromatographie: 935 mg 1B, Schaum

C. 3,3-[2,2-Dimethyl-1,3-propandiylbis(oxy)]-10β-[[3-(methylamino)phenyl]thio]-9(11)-estr-en-5α,17β-diol (1C)

9,4 g 1B und 2,5 g Lithiumaluminiumhydrid werden in 400 ml THF abs. bei 80 °C unter Argon 1 Stunde am Rückfluß erhitzt.
Anschließend wird überschüssiges Lithiumaluminiumhydrid mit Wasser hydrolysiert, mit Essigester verdünnt, die org. Phase abgetrennt und mit Wasser gewaschen, über $Na_2SO_4$ getrocknet und das Lösungsmittel am Rotationsverdampfer abgezogen.
Ausbeute nach Säulenchromatographie: 8,4 g 1C, Schaum

D. 3,3-[2,2-Dimethyl-1,3-propandiylbis(oxy)]-10β-[[3-(N-formyl-N-methylamino)phenyl] thio]-9(11)-estr-en-5α,17β-diol (1D)

Durchführung und Aufarbeitung wie Beispiel 1B.
Eingesetzte Mengen: 8,6 g 1C, 3,39 ml Acetanhydrid, 1,4 ml Ameisensäure, 45 ml Pyridin p.a.
Ausbeute nach Säulenchromatographie: 6,0 g 1D, Schaum

E. 10β-[[3-(Dimethylamino)phenyl]thio]-3,3-[2,2-dimethyl-1,3-propandiylbis(oxy)]-9(11)-estr-en-5α,17β-diol (1E)

3,95 g 1D werden in 150 ml abs. THF gelöst und bei 0°C unter Argon mit 1,46 ml einer 10 molaren Borandimethylsulfidkomplex-Lösung in THF versetzt. Es wird 2,5 Stunden bei Raumtemperatur nachgerührt. Das Reaktionsgemisch wird bei 0°C langsam mit 4 ml Methanol versetzt und über Nacht an der Luft stehen gelassen. Anschließend wird mit $H_2O$ und Essigester verdünnt. Die organische Phase wird abgetrennt, mit $H_2O$ gewaschen, über $Na_2SO_4$ getrocknet und im Vakuum eingeengt. Ausbeute nach Säulenchromatographie: 3,6 g 1E, Schaum.

F. 10β-[[2-Brom-5-(dimethylamino)phenyl]thio]-3,3-[2,2-dimethyl-1,3-propandiylbis(oxy)]-9(11)-estr-en-5α,17β-diol (1F)

5 g 1E werden in 200 ml $CCl_4$ gelöst, bei 0 °C mit 1,65 g N-Bromsuccinimid versetzt und bei 0 °C unter Argon 2 Stunden gerührt. Das Reaktionsgemisch wird mit $CH_2Cl_2$ verdünnt und mit gesättigter $NaHCO_3$-Lösung versetzt, die org. Phase mit Wasser gewaschen und über $Na_2SO_4$ getrocknet und das Lösungsmittel am Rotationsverdampfer abgezogen.
Ausbeute nach Säulenchromatographie: 2,7 g 1F, Schaum

G. 10β,11β-[5-(Dimethylamino)-o-phenylenthio]-3,3-[2,2-dimethyl-1,3-propandiylbis(oxy)]estran-5α,17β-diol (1G)

1,7 g 1F, 1,15 ml Tri-n-butylzinnhydrid und 30 mg Azobisisobutyronitril werden in 115 ml Toluol p.a. unter Argon und Rückfluß 1 Stunde lang im Licht einer 300W-Glühbirne gerührt.
Säulenchromatographie ergibt 675 mg 1G als Schaum.

H. 11β-[4-(Dimethylamino)-2-(methylthio)-phenyl]-3,3-[2,2-dimethyl-1,3-propandiylbis(oxy)]-5(10)-estren-17β-ol (1H)

300 ml flüssiger Ammoniak wird in einen auf -70°C gekühlten Kolben einkondensiert. Eine Lösung von 7,23 g 1G in 50 ml absolutem Tetrahydrofuran wird bei -70°C zugetropft. 521 mg Lithium werden portionsweise zugegeben, dann wird 3 Stunden unter Schutzgas bei derselben Temperatur gerührt. Anschließend werden 1,5 ml t-Butanol zugetropft und weitere 2 Stunden gerührt. Nach Zugabe von 1,5 ml Iodmethan wird auf Raumtemperatur erwärmt und mit Wasser verdünnt. Die Mischung wird mit Ethylacetat extrahiert. Die organischen Phasen werden mit Wasser gewaschen, über Natriumsulfat getrocknet und im Vakuum eingedampft. Säulenchromatographie des Rückstandes ergibt 4,34 g 1H als weißen Schaum.

I. 11β-[4-(Dimethylamino)-phenyl]-3,3-[2,2-dimethyl-1,3-propandiylbis(oxy)]-5(10)-estren-17β-ol (1I)

Vier Spatelspitzen Raney-Nickel werden fünfmal mit Methanol gewaschen und mit einer Lösung von 4,30 g 1H in 200 ml Methanol versetzt. Das Reaktionsgemisch wird 16 Stunden unter Schutzgas bei Raumtemperatur gerührt. Die Lösung wird abdekantiert, der Rückstand wird zweimal mit Methanol und dreimal mit Methylenchlorid gewaschen. Die vereinigten Lösungen werden über eine Fritte abgesaugt und im Vakuum eingeengt. Säulenchromatographie des Rückstandes ergibt 2,35 g 1I als weißen Schaum.

K. 11β-[4-(Dimethylamino)phenyl]-3,3-[2,2-dimethyl-1,3-propandiylbis(oxy)]-5(10)-estren-17-on (1K)

Eine Lösung von 2,3 g 1I, 2,45 ml Cyclohexan und 350 mg Aluminiumtriisopropylat in 50 ml Toluol wird 14 Stunden unter Schutzgas am Wasserabscheider gekocht. Das Reaktionsgemisch wird nach dem Abkühlen mit Wasser versetzt und mit Ethylacetat extrahiert. Die organische Phase wird mit gesättigter Natriumchloridlösung gewaschen, über Natriumsulfat getrocknet und am Vakuum eingeengt. Der Rückstand wird an Aluminiumoxid (neutral, Stufe III) mit einem Gemisch aus Hexan/Ethylacetat chromatographiert. Man erhält 2,16 g 1K als weißen Schaum.

L. 11β-[4-(Dimethylamino)phenyl]-3,3-[2,2-dimethyl-1,3-propandiylbis(oxy)]-17α-(1-propinyl)-5(10)-estren-17β-ol (1L)

75 ml absolutes Tetrahydrofuran werden durch einstündiges Einleiten von Propin bei 0°C gesättigt. Anschließend werden bei 0°C 16 ml einer 15%igen Lösung von Butyllithium in Hexan zugetropft und eine Stunde unter Schutzgas gerührt. Nach Zutropfen einer Lösung von 2,1 g 1K wird 20 Stunden bei Raumtemperatur gerührt. Das Reaktionsgemisch wird mit Wasser versetzt und mit Ethylacetat extrahiert. Die vereinigten organischen Phasen werden mit gesättigter Natriumchloridlösung gewaschen, über Natriumsulfat getrocknet und am Vakuum eingeengt. Der Rückstand wird an Aluminiumoxid (neutral, Stufe III) mit einem Gemisch aus Hexan/Ethylacetat chromatographiert. Es werden 2,13 g 1L als weißer Schaum erhalten.

M. 11β-[4-(Dimethylamino)phenyl]-17β-hydroxy-17α-(1-propinyl)-5(10)-estren-3-on (1M)

2,11 g 1L werden in 100 ml Aceton gelöst, mit 17 ml 4 n wäßriger Salzsäure versetzt und 30 Minuten unter Schutzgas bei Raumtemperatur gerührt. Die Reaktionslösung wird mit gesättigter Natriumhydrogencarbonatlösung neutralisiert und mit Ethylacetat extrahiert. Die vereinigten organischen Phasen werden mit gesättigter Natriumchloridlösung gewaschen, über Natriumsulfat getrocknet und am Vakuum eingeengt. Säulenchromatographie des Rückstandes ergibt 1,59 g 1M als weißen Schaum.

N. 11β-[4-(Dimethylamino)phenyl)]-17β-hydroxy-17α-(1-propinyl)-4-estren-3-on (1N)

509 mg p-Toluolsulfonsäure werden in 95 ml Toluol unter Schutzgas zum Sieden erhitzt. 1,05 g 1M, gelöst in 5 ml Toluol werden schnell zugegeben. Es wird 45 Minuten bei 110°C gerührt, dann werden einige Tropfen Triethylamin zugegeben. Nach dem Abkühlen wird mit Natriumhydrogencarbonatlösung gewaschen. Die organische Phase wird über Natriumsulfat getrocknet und am Vakuum eingeengt. Säulenchromatographie ergibt 223 mg der Titelverbindung 1N als weißen Schaum.
$^1$H-NMR($CDCl_3$) δ:7,27 ppm(2H,d J=8Hz,aromatisch); 6,67 ppm(2H,d J=8Hz, aromatisch); 5,85 ppm(IH,s breit,H-4);

3,33 ppm(1H,dd breit J=6,0Hz und J=5,5Hz, H11) 2,94ppm(6H,s,H-$NCH_3$); 1,89ppm(3H,s,H-$CH_3$-C≡C-); 0,67ppm(3H,s,H-18). $[\alpha]_d^{20}$=17,0°($CHCl_3$; c=0,505).

Die Verbindung 1I läßt sich auch nach folgender Vorschrift herstellen:

Zu einer Lösung von 2,5 g 11β-[4-(Dimethylamino)phenyl]-17β-hydroxy-5(10)-estren-3-on (Herstellung siehe Tet.Let 24, 5205 (1983)) in 18 ml Methylenchlorid werden 1,66 g 2,2-Dimethyl-1,3-propandiol, 0,87 ml Trimethoxymethan und 150 mg p-Toluolsulfonsäure gegeben. Die Reaktionsmischung wird 14 Stunden unter Schutzgas bei Raumtemperatur gerührt, mit Methylenchlorid verdünnt und mit Natriumhydrogencarbonatlösung gewaschen. Die wäßrige Phase wird mit Methylenchlorid extrahiert. Die vereinigten organischen Phasen werden über Natriumsulfat getrocknet und am Vakuum eingeengt. Säulenchromatographie des Rückstandes ergibt 2,32 g 1I.

**BEISPIEL 2**

Herstellung von 3,3-[2,2-Dimethyl-1,3-propandiylbis(oxy)]-10β,11β-(5-methoxy-o-phenylenthio)-estran-5α,17β-diol (2B)

A. 10β-[(2-Brom-5-methoxyphenyl)thio]-3,3-[2,2-dimethyl-1,3-propandiylbis(oxy)]9(11)-estr-en-5α,17β-diol (2A)

Die Durchführung und Aufarbeitung erfolgt wie in Beispiel 1A beschrieben. Eingesetzte Mengen: 12,73 g 3,3-[2,2-Dimethyl-1,3-propandiylbis(oxy)]-5α,10α-epoxy-estr-9(11)-en-17β-ol in 100 ml abs. Tetrahydrofuran, 22,3 g 2-Brom-5-methoxythiophenol in 182 ml abs. Tetrahydrofuran, 40 ml 2,5 molarer-Butyllithiumlösung in n-Hexan. Säulenchromatographie ergibt 17,99 g 2A, Fp.: 117-119°C.

B. 3,3-[2,2-Dimethyl-1,3-propandiylbis(oxy)]-10β,11β-(5-methoxy-o-phenylenthio)-estran-5α,17β-diol (2B)

Durchführung und Aufarbeitung erfolgt wie in Beispiel 1G.
Ausbeute: 1,09 g 2B, Fp.: 95-97°C.

**BEISPIEL 3**

Herstellung von 11β-[4-(Dimethylamino)phenyl]-17β-hydroxy-17α-(3-hydroxy-1Z-propenyl)-4-estren-3-on (3E)

A. 11β-[4-(Dimethylamino)phenyl]-17β-hydroxy-4-estren-3-on (3A)

Eine Lösung von 2,7 g 11β-[4-(Dimethylamino)phenyl]-17β-hydroxy-5(10)-estren 3-on (Herstellung siehe Tet.Let. 24, 5205 (1983)) in 270 ml Methylenchlorid wird mit 3,91 g p-Toluolsulfonsäure einen Tag unter Schutzgas zum Sieden erhitzt, dann werden einige Tropfen Triethylamin zugegeben. Nach dem Abkühlen wird mit Natriumhydrogencarbonatlösung gewaschen. Die organische Phase wird über Natriumsulfat getrocknet und am Vakuum eingeengt. Säulenchromatographie ergibt 1,85 g 3A als weißen Schaum.

B. 11β-[4-(Dimethylamino)phenyl]-3,3-[1,2-ethandiylbis(thio)]-4-estren-17β-ol (3B)

Zu einer Lösung von 500 mg 3A in 5 ml Eisessig werden 0,11 ml 1,2-Ethandithiol und 3,62 mg p-Toluolsulfonsäure gegeben. Das Reaktionsgemisch wird eine Stunde bei Raumtemperatur unter Schutzgas gerührt und dann mit 2n wäßriger Natronlauge basisch gemacht. Die wäßrige Phase wird mit Ethylacetat extrahiert. Die vereinigten organischen Phasen werden mit gesättigter Natriumhydrogencarbonatlösung gewaschen, über Natriumsulfat getrocknet und am Vakuum eingeengt. Säulenchromatographie ergibt 399 mg 3B.

C. 11β-[4-(Dimethylamino)phenyl]-3,3-[1,2-ethandiylbis(thio)]-4-estren-17-on (3C)

Eine Lösung von 390 mg 3B, 0,42 ml Cyclohexanon und 76 mg Aluminiumtriisopropylat in 10 ml Toluol wird 30 Stunden unter Schutzgas am Wasserabscheider gekocht. Nach dem Abkühlen wird das Reaktionsgemisch mit Wasser versetzt und mit Ethylacetat extrahiert. Die organische Phase wird mit gesättigter Natriumchloridlösung gewaschen, über Natriumsulfat getrocknet und am Vakuum eingeengt. Säulenchromatographie ergibt 330 mg 3C als weißen Schaum.

D. 11β-[4-(Dimethylamino)phenyl]-3,3-[1,2-ethandiylbis(thio)]-17α-[3-[(tetrahydro-2H-pyran-2-yl)oxyl]-1-propinyl]-4-estren-17β-ol (3D)

Eine Lösung von 0,89 ml 2-(2-Propinyloxy)tetrahydro-2H-pyran in 30 ml Tetrahydrofuran wird bei 0°C mit einer Lösung von 3,66 ml einer 15%igen Lösung von Butyllithium in Hexan versetzt und 30 Minuten bei 0°C unter Schutzgas gerührt. Bei derselben Temperatur wird dann eine Lösung von 280 mg 3C in 10 ml Tetrahydrofuran zugetropft. Anschließend wird zwei Stunden bei 0°C gerührt. Das Reaktionsgemisch wird mit Ammoniumchloridlösung versetzt und it Ethylacetat extrahiert. Die vereinigten organischen Phasen werden mit Natriumchloridlösung gewaschen, über Natriumsulfat getrocknet und am Vakuum eingeengt. Säulenchromatographie ergibt 328 mg 3D als weißen Schaum.

E. 11β-[4-(Dimethylamino)-phenyl]-17β-hydroxy-17α-(3-hydroxy-1-propinyl)-4-estren-3-on (3E)

300 mg 3D sowie 2,21 g Glyoxylsäure-Hydrat werden in 18 ml Eisessig gelöst. Das Reaktionsgemisch wird 1,5 Minuten unter Schutzgas bei Raumtemperatur gerührt, mit 2,1 ml 4 n wäßriger Salzsäure versetzt und weitere 10 Minuten gerührt. Dann wird mit Natriumhydrogencarbonatlösung neutralisiert und mit Ethylacetat extrahiert. Die vereinigten organischen Phasen werden mit Natriumchloridlösung gewaschen, über Natriumsulfat getrocknet und am Vakuum eingeengt. Säulenchromatographie liefert 135 mg 3E.
Kristallisation aus Ethylacetat/Hexan führt zu 112 mg der genannten Verbindung. Fp.: 142-145°C; $[\alpha]_D^{20}$ +4,0°($CHCl_3$; c=1,000)

F. 11β-[4-(Dimethylamino)phenyl]-17β-hydroxy-17α-(3-hydroxy-1Z-propenyl)-4-estren-3-on (3F)

Eine Lösung von 78 mg 3E in 2 ml Ethanol und 2 ml Pyridin wird in Gegenwart von 13 mg 10%igem Pd/$BaSO_4$-Katalysator 3 Stunden hydriert. Das Reaktionsgemisch wird über Celite abgesaugt und mit Ethanol und Methylenchlorid nachgewaschen. Das Filtrat wird am Vakuum eingeengt. Den Rückstand chromatographiert man an Kieselgel mit einem Gemisch aus Methylenchlorid/Methanol. Es werden 63 mg 3F als weißer Schaum isoliert.
$^1$H-NMR($CD_2Cl_2$) δ: 7,27 ppm (2H,d J=9Hz,H-aromatisch); 6,67 ppm (2H,d J=9Hz,Haromatisch); 5,78 ppm(1H,s breit,H-4); 5,68 ppm(1H,ddd J=12,5Hz und J=5,0Hz und J=4,5Hz,H-CH=); 5,63 ppm(1H,d J=12,5Hz,H-CH=); 4,32 ppm(1H,dd J=13,0Hz und J=5,0Hz); 3,28 ppm(1H,dd breit J=6,0Hz und J=5,0Hz,H-11); 2,92 ppm(6H,s,H-$NCH_3$); 0,71 ppm(3H,s,H-18).

**BEISPIEL 4**

Herstellung von 11β-(4-Acetylphenyl)-17β-hydroxy-17α-(1-propinyl)-4-estren-3-on (4K)

A. 17β-Hydroxy-11β-(4-hydroxyphenyl)-5(10)-estren-3-on (4A)

Zu einer Lösung von 5 g 17β-Hydroxy-11β-(4-methoxyphenyl)-5(10)-estren-3-on (Herstellung siehe Tet.Let. 24,5205(1983)) in 50 ml abs. N,N-Dimethylformamid werden 3,27 g Natriummethanthiolat gegeben. Das Reaktionsgemisch wird 90 Minuten bei 160°C gerührt. Nach dem Abkühlen wird mit Wasser verdünnt und mit Ethylacetat extrahiert. Die organische Phase wird mit gesättigter Natriumhydrogencarbonatlösung gewaschen, über Natriumsulfat getrocknet und am Vakuum eingeengt. Säulenchromatographie ergibt 4,29 g 4A als weißen Schaum.

B. 3,3-[2,2-Dimethyl-1,3-propandiylbis(oxy)]-11β-(4-hydroxyphenyl)-5(10)-estren 17β-ol (4B)

Eine Lösung von 4,2 g 4A, 1,72 g 2,2-Dimethyl-1,3-propandiol, 1,5 ml Trimethoxymethan und 250 mg p-Toluolsulfonsäure in 40 ml Methylenchloride wird 14 Stunden unter Schutzgas bei Raumtemperatur gerührt. Dann wird gesättigte Natriumhydrogencarbonatlösung zugegeben. Die wäßrige Phase wird mit Methylenchlorid extrahiert. Die vereinigten organischen Phasen werden über Natriumsulfat getrocknet und am Vakuum eingeengt. Säulenchromatographie führt zu 3,25 g 4B als weißer Schaum.

C. 3,3-[2,2-Dimethyl-1,3-propandiylbis(oxy)]-11β-[4-[[(trifluormethyl)sulfonyl]oxy]phenyl]-5(10)-estren 17β-ol (4C)

Zu einer Lösung von 3,2 g 4B und 4,75 4-(Dimethylamino)-pyridin in 100 ml Methylenchlorid wird bei -78°C unter Schutzgas eine Lösung von 1,86 ml Trifluormethansulfonsäureanhydrid in 10 ml Methylenchlorid getropft. Das Reaktionsgemisch wird 2 Stunden bei -78°C nachgerührt und dann in gesättigte Natriumhydrogencarbonatlösung gegossen. Die wäßrige Phase wird mit Methylenchlorid extrahiert. Die vereinigten organischen Phasen werden mit Wasser gewaschen, über Natriumsulfat getrocknet und am Vakuum eingeengt. Säulenchromatographie ergibt 2,9 g 4C als weißen Schaum.

D. 11β-(4-Acetylphenyl)-3,3-[2,2-Dimethyl-1,3-propandiylbis(oxy)]-5(10)-estren-17β-ol (4D)

Eine Lösung von 2,84 g 4C in 35 ml N,N-Dimethylformamid wird mit 393 mg Lithiumchlorid versetzt und 15 Minuten unter Schutzgas bei Raumtemperatur gerührt. Dann werden 277 mg Tetrakis(triphenylphosphin)palladium(0) und 1,7 ml (1-Ethoxyethenyl)tributylstannan zugegeben. Das Reaktionsgemisch wird 3 Stunden bei 110°C gerührt, nach dem Erkalten mit 120 ml Ethylacetat verdünnt und über Celite abgesaugt. Das Filtrat wird viermal mit gesättigter Natriumchloridlösung gewaschen, über Natriumsulfat getrocknet und am Vakuum eingeengt. Säulenchromatographie ergibt 1,79 g 4D, weißer Schaum.

E. 11β-(4-Acetylphenyl)-17β-hydroxy-5(10)-estren-3-on (4E)

1,7 g 4D werden in 100 ml Aceton gelöst, mit 5,4 ml 4 n wäßriger Salzsäure versetzt und eine Stunde unter Schutzgas bei Raumtemperatur gerührt. Die Reaktionslösung wird mit gesättigter Natriumhydrogencarbonatlösung neutralisiert und mit Ethylacetat extrahiert. Die vereinigten organischen Phasen werden mit gesättigter Natriumchloridlösung gewaschen, über Natriumsulfat getrocknet und am Vakuum eingeengt. Säulenchromatographie ergibt 1,31 g 4E, weißer Schaum.

F. 11β-(4-Acetylphenyl)-17β-hydroxy-4-estren-3-on (4F)

485 mg p-Toluolsulfonsäure werden in 100 ml Toluol unter Schutzgas auf 90°C erhitzt. 500 mg 4E, gelöst in 5 ml Toluol werden schnell zugegeben. Das Reaktionsgemisch wird eine Stunde bei 90°C gerührt und nach dem Abkühlen mit gesättigter Natriumhydrogencarbonatlösung versetzt. Die wäßrige Phase wird mit Ethylacetat extrahiert. Die vereinigten organischen Phasen werden über Natriumsulfat getrocknet und am Vakuum eingeengt. Der Rückstand wird an Kieselgel mit Ethylacetat/Hexan chromatographiert. Man erhält 239 mg der Titelverbindung 4F als weißen Schaum.
$^1$H-NMR($CDCl_3$) δ: 7,90 ppm (2H,d J=8Hz,H-aromatisch); 7,54 ppm (2H,d J=8Hz, H-aromatisch); 5,87 ppm (1H,s,H-4); 3,61 ppm (1H,dd breit J=9,0Hz und J=7,5Hz,H-17); 3,42 ppm (1H,dd breit J=6,0Hz und J=5,5Hz,H-11); 2,61 ppm (3H,s,H-Ac); 0,52 ppm (3H,s,H-18).

G. 3,3-[1,2-Ethandiylbis(thio)]-11β-[4-(2-methyl-1,3-dithiolan-2-yl)phenyl]-4-estren-17β-ol (4G)

Zu einer Lösung von 2,12 g 4F in 40 ml Eisessig werden 0,90 ml 1,2-Ethandithiol und 1,18 g *p*-Toluolsulfonsäure gegeben. Das Reaktionsgemisch wird fünf Stunden bei Raumtemperatur unter Schutzgas gerührt und dann mit 2 M wäßriger Natronlauge basisch gemacht. Die wäßrige Phase wird mit Ethylacetat extrahiert. Die vereinigten organischen Phasen werden mit gesättigter Natriumhydrogencarbonatlösung gewaschen, über Natriumsulfat getrocknet und am Vakuum eingeengt. Das Rohprodukt wird in 80 ml Methylenchlorid und 160 ml Ethanol gelöst und mit 1,49 g festem Kaliumcarbonat sechs Stunden am Rückfluß erhitzt. Die erkaltete Lösung wird filtriert und eingeengt. Säulenchromatographie ergibt 2,02 g 4G als weißen Schaum.

H. 3,3-[1,2-Ethandiylbis(thio)]-11β-[4-(2-methyl-1,3-dithiolan-2-yl)phenyl]-4-estren-17-on (4H)

Wie unter 3C beschrieben werden aus 1,95 g 4G mit 2,42 ml Cyclohexanon und 438 mg Aluminiumtriisopropylat in 35 ml Toluol 1,81 g 4H hergestellt.
IR (KBr): 1740 $cm^{-1}$ (C=O).

I. 3,3-[1,2-Ethandiylbis(thio)]-11β-[4-(2-methyl-1,3-dithiolan-2-yl)phenyl]-17α-(1-propinyl)-4-estren-17β-ol (4I)

Wie unter 1L beschrieben werden aus 763 mg 4H mit 5,8 ml einer 15%igen Lösung von Butyllithium in Hexan in 50 ml mit Propin gesättigtem Tetrahydrofuran 779 mg 4I hergestellt.
IR (KBr): 2240 $cm^{-1}$ (C≡C).

K. 11β-(4-Acetylphenyl)-17β-hydroxy-17α-(1-propinyl)-4-estren-3-on (4K)

1,28 g *N*-Chlorsuccinimid und 1,83 g Silber(I)nitrat werden in 50 ml eines Acetonitril/Wasser-Gemisches (8 : 2) vorgelegt. Bei Raumtemperatur wird unter Schutzgas eine Lösung von 700 mg 4I in 3 ml Aceton und 2 ml Acetonitril zügig zugetropft. Nach 15 Minuten wird nacheinander mit gesättigter Natriumsulfitlösung, gesättigter Natriumcarbonatlösung und gesättigter Natriumchloridlösung sowie Methylenchlorid versetzt. Das Gemisch wird über Celite filtriert, über Natriumsulfat getrocknet und am Vakuum eingeengt. Säulenchromatographie liefert 238 mg der Titelverbindung 4K als blaßgelben Schaum.
IR (KBr); 2235 $cm^{-1}$ (C≡C), 1678 $cm^{-1}$ (C=O), 1664 $cm^{-1}$ (C=O).

$^1$H-NMR($CDCl_3$) δ: 7,90 ppm (2H,d J=9 Hz,H-aromatisch); 7,54 ppm (2H,d J=9 Hz,H-aromatisch); 5,88 ppm (1H,s breit,H-4); 3,48 ppm (1H,dd breit J=6,5 Hz und J=6,0 Hz,H-11); 2,80 ppm (1H,m,H-10); 2,60 ppm (3H,s,H-Ac); 1,89 ppm (3H,s,H-$CH_3$-C≡C-); 0,59 ppm (3H,s,H-18).

**BEISPIEL 5**

Herstellung von (*Z*)-11β-(4-Acetylphenyl)-17β-hydroxy-17α-(3-hydroxy-1-propenyl)-4-estren-3-on(5C)

A. 3,3-[1,2-Ethandiylbis(thio)]-11β-[4-(2-methyl-1,3-dithiolan-2-yl)phenyl]-17α-[3-[(tetrahydro-2*H*-pyran-2-yl)oxy]-1-propinyl-4-estren-17β-ol (5A)

Wie unter 3D beschrieben werden aus 1,0 g 4H mit 2,74 ml 2-(2-Propinyloxy)tetrahydro-2*H*-pyran und 11,3 ml einer 15%igen Lösung von Butyllithium in Hexan in 120 ml Tetrahydrofuran 1,19 g 5A hergestellt.

B. 11β-(4-Acetylphenyl)-17β-hydroxy-17α-(3-hydroxy-1-propinyl)-4-estren-3-on (5B)

Wie unter 4K beschrieben werden 1,73 g 5A mit 2,71 g *N*-Chlorsuccinimid und 3,87 g Silber(I)nitrat in 10 ml Aceton, 24 ml Wasser und 96 ml Acetonitril umgesetzt. Das Rohprodukt wird mit 127 mg Pyridinium *p*-toluolsulfonat in 15 ml feuchtem Ethanol zwei Stunden bei 60°C gerührt. Die Lösung wird auf das halbe Volumen eingedampft, mit Ethylacetat verdünnt, mit halbgesättigter Natriumchloridlösung und mit Wasser gewaschen, über Natriumsulfat getrocknet und am Vakuum eingeengt. Säulenchromatographie ergibt 265 mg 5B als blaßgelben Schaum.
IR (KBr): 2235 $cm^{-1}$ (C≡C), 1678 $cm^{-1}$ (C=O), 1665 $cm^{-1}$ (C=O).
$^1$H-NMR($CDCl_3$) δ: 7,91 ppm (2H,d J=9 Hz,H-aromatisch); 7,54 ppm (2H,d J=9 Hz,H-aromatisch); 5,88 ppm (1H,s breit,H-4); 4,37 ppm (2H,s breit,H-$CH_2OH$); 3,48 ppm (1H,dd breit J=6,0 Hz und J=5,0 Hz,H-11); 2,81 ppm (1H,m,H-10); 2,61 ppm (3H,s,H-Ac); 0,62 ppm (3H,s,H-18).

C. (*Z*)-11β-(4-Acetylphenyl)-17β-hydroxy-17α-(3-hydroxy-1-propenyl)-4-estren-3-on (5C)

Wie unter 3F beschrieben werden 245 mg 5B mit 60 mg 10%igem Pd/$BaSO_4$-Katalysator in 4 ml Pyridin und 4 ml Ethanol hydriert. Nach Kieselgelchromatographie werden 170 mg der Titelverbindung 5C erhalten. Kristallisation aus Methylenchlorid / Diisopropylether liefert 146 mg 5C als hellgelbe Kristalle.
Fp.: 225-230°C; $[\alpha]_D^{22}$=+76,4° ($CHCl_3$; c=0,500).
IR (KBr): 1680 $cm^{-1}$ (C=O), 1665 $cm^{-1}$ (C=O).

**BEISPIEL 6**

Herstellung von 11β-[4-(Dimethylamino)phenyl]-17α-(1-propinyl)-4-estren-17β-ol (6C)

A. 11β-[4-(Dimethylamino)phenyl]-4-estren-17β-ol (6A)

120 ml flüssiger Ammoniak wird in einen auf -75°C gekühlten Kolben einkondensiert. 91 mg Lithium werden zugegeben. Nach 20 Minuten wird eine Lösung von 770 mg 3B in 4 ml Tetrahydrofuran zugetropft. 182 mg Lithium werden portionsweise zugegeben, dann wird zwei Stunden bei -75°C gerührt. Anschließend werden 9 ml Ethanol zugetropft. Der Ansatz wird bei Raumtemperatur über Nacht stehengelassen, dann mit Wasser verdünnt. Die Mischung wird mit Ethylacetat extrahiert. Die organischen Phasen werden mit gesättigter Natriumchloridlösung gewaschen, über Natriumsulfat getrocknet und im Vakuum eingeengt. Kieselgelchromatographie des Rückstands ergibt 350 mg 6A als weißen Schaum.
$^1$H-NMR($CD_2Cl_2$) δ: 7,27 ppm (2H,d J=9 Hz,H-aromatisch); 6,63 ppm (2H,d J=9 Hz,H-aromatisch); 5,40 ppm (1H,s breit,H-4); 3,51 ppm (1H,dd breit J=9 Hz und J=6 Hz,H-17); 3,22 ppm (1H,dd breit J=6 Hz und J=5 Hz,H-11); 2,91 ppm (6H,s,H-$NCH_3$); 2,46 ppm (1H,m,H-10); 0,50 ppm (3H,s,H-18).

B. 11β-[4-(Dimethylamino)phenyl]-4-estren-17-on (6B)

Wie unter 3C beschrieben werden aus 327 mg 6A mit 400 µl Cyclohexanon und 409 mg Aluminiumtriisopropylat in 10 ml Toluol 225 mg 6B hergestellt.
IR (KBr): 1740 $cm^{-1}$ (C=O).
$^1$H-NMR($CD_2Cl_2$) δ: 7,28 ppm (2H,d J=9 Hz,H-aromatisch); 6,64 ppm (2H,d J=9 Hz,H-aromatisch); 5,43 ppm (1H,s

breit,H-4); ; 3,29 ppm (1H,dd breit J=5,5 Hz und J=5 Hz,H-11); 2,91 ppm (6H,s,H-$NCH_3$); 2,50 ppm (1H,m,H-10); 0,63 ppm (3H,s,H-18).

C. 11β-[4-(Dimethylamino)phenyl]-17α-(1-propinyl)-4-estren-17β-ol (6C)

Wie unter 1L beschrieben werden aus 215 mg 6B mit 2,1 ml einer 15%igen Lösung von Butyllithium in Hexan in 20 ml mit Propin gesättigtem Tetrahydrofuran 207 mg 6C hergestellt. Kristallisation aus Pentan / Ethylacetat führt zu 168 mg der genannten Verbindung.
Fp.: 187°C; $[\alpha]_D^{22}$=-28,3° ($CHCl_3$; c=0,505).
IR (KBr): 2240 $cm^{-1}$ (C≡C).

**BEISPIEL 7**

Herstellung von (*Z*)-17β-Hydroxy-17α-(3-hydroxy-1-propenyl)-11β-[4-(3-pyridinyl)phenyl]-4-estren-3-on (7I)

A. 3,3-[2,2-Dimethyl-1,3-propandiylbis(oxy)]-11β-[4-(3-pyridinyl)phenyl]-5(10)-estren-17β-ol (7A) und

B. 3,3-[2,2-Dimethyl-1,3-propandiylbis(oxy)]-11β-(4-ethylphenyl)-5(10)-estren-17β-ol (7B)

Unter Schutzgas werden zu einer Lösung von 2,71 g 4C in 40 ml Toluol und 18 ml Ethanol 747 mg Diethyl(3-pyridinyl)boran (Aldrich), 392 mg Lithiumchlorid, 6 ml 2 M Natriumcarbonatlösung und 267 mg Tetrakis(triphenylphosphin)palladium(0) gegeben. Das Gemisch wird zwei Stunden auf 95°C erhitzt und nach dem Abkühlen in Ethylacetat aufgenommen. Die organische Phase wird mit gesättigter Natriumchloridlösung gewaschen, über Natriumsulfat getrocknet und im Vakuum eingeengt. Nach Säulenchromatographie werden 1,94 g 7A sowie 171 mg 7B erhalten.

C. 17β-Hydroxy-11β-[4-(3-pyridinyl)phenyl]-5(10)-estren-3-on (7C)

Wie unter 4E beschrieben werden aus 1,9 g 7A mit 7 ml 4 M wäßriger Salzsäure in 150 ml Aceton 1,07 g 7C hergestellt.

D. 17β-Hydroxy-11β-[4-(3-pyridinyl)phenyl]-4-estren-3-on (7D)

1,06 g 7C werden in 20 ml Chloroform gelöst, unter Schutzgas mit 944 mg *p*-Toluolsulfonsäure versetzt und drei Stunden am Rückfluß erhitzt. Die erkaltete Lösung wird mit einigen Tropfen Triethylamin versetzt, mit Methylenchlorid verdünnt, mit gesättigter Natriumchloridlösung gewaschen, über Natriumsulfat getrocknet und am Vakuum eingedampft. Kieselgelchromatographie ergibt 644 mg 7D.
IR (KBr): 1667 $cm^{-1}$ (C=O).
$^1$H-NMR($CDCl_3$) δ: 8,87 ppm (1H,d breit J=2 Hz,H-Py2); 8,58 ppm (1H,dd breit J=5,0 Hz und J=2 Hz,H-Py6); 7,89 ppm (1H,ddd J=8 Hz und J=2 Hz und J=2 Hz,H-Py4); 7,54 ppm (4H,m,H-aromatisch); 7,37 ppm (1H,dd breit J=8 Hz und J=5,0 Hz,H-Py5); 5,88 ppm (1H,s breit,H-4); 3,61 ppm (1H,dd breit J=9,0 Hz und J=7,0 Hz,H-17); 3,40 ppm (1H,dd breit J-6,0 Hz und J=5,0 Hz,H-11); 2,86 ppm (1H,m,H-10); 0,58 ppm (3H,s,H-18).

E. 3,3-[1,2-Ethandiylbis(thio)]-11β-[4-(3-pyridinyl)phenyl]-4-estren-17β-ol (7E)

Wie unter 4G beschrieben werden aus 629 mg 7D mit 123 µl 1,2-Ethandithiol und 419 mg *p*-Toluolsulfonsäure in 6 ml Eisessig sowie mit 203 mg Kaliumcarbonat in 8 ml Methylenchlorid und 45 ml Methanol 569 mg 7E hergestellt.

F. 3,3-[1,2-Ethandiylbis(thio)]-11β-[4-(3-pyridinyl)phenyl]-4-estren-17-on (7F)

Wie unter 3C beschrieben werden aus 550 mg 7E mit 510 µl Cyclohexanon und 100 mg Aluminiumtriisopropylat in 17 ml Toluol 460 mg 7F hergestellt.
IR (KBr): 1736 $cm^{-1}$ (C=O).

G. 3,3-[1,2-Ethandiylbis(thio)]-11β-[4-(3-pyridinyl)phenyl-17α-[3-[(tetrahydro-2*H*-pyran-2-yl)oxy]-1-propinyl]-4-estren-17β-ol (7G) (7G)

Wie unter 3D beschrieben werden aus 442 mg 7F mit 1,30 ml 2-(2-Propinyloxy)tetrahydro-2*H*-pyran und 5,36 ml einer 15%igen Lösung von Butyllithium in Hexan in 70 ml Tetrahydrofuran 460 mg 7G hergestellt.

H. 17β-Hydroxy-17α-(3-hydroxy-1-propinyl)-11β-[4-(3-pyridinyl)phenyl]-4-estren-3-on (7H)

Wie unter 3E beschrieben werden aus 453 mg 7G mit 3,27 g Glyoxylsäure und 2,98 ml 4 M wäßriger Salzsäure in 8 ml Aceton und 8 ml Eisessig 234 mg 7H hergestellt.
$^1$H-NMR($CDCl_3$) δ: 8,88 ppm (1H, d breit J=2 Hz,H-Py2); 8,59 ppm (1H, dd breit J=4,5 Hz und J=2 Hz,H-Py6); 7,90 ppm (1H,ddd J=8 Hz und J=2 Hz und J=2 Hz,H-Py4); 7,53 ppm (4H,m,H-aromatisch); 7,38 ppm (1H,dd breit J=8 Hz und J=45, Hz,H-Py5); 5,89 ppm (1H,s breit,H-4); 4,38 ppm (2H,m,H-$CH_2OH$); 3,48 ppm (1H,dd breit J=6,0 Hz und J=5,0 Hz,H-11); 2,88 ppm (1H,m,H-10); 0,68 ppm (3H,s,H-18).

I. (*Z*)-17β-Hydroxy-17α-(3-hydroxy-1-propenyl)-11β-[4-(3-pyridinyl)phenyl]-4-estren-3-on (7I)

Wie unter 3F beschrieben werden 225 mg 7H mit 45 mg 10%igem Pd/$BaSO_4$-Katalysator in 2 ml Pyridin und 4 ml Ethanol hydriert. Nach Kieselgelchromatographie werden 160 mg der Titelverbindung 7I erhalten.
IR (KBr): 1663 $cm^{-1}$ (C=O).
$^1$H-NMR($CDCl_3$) δ: 8,88 ppm (1H, d breit J=2 Hz,H-Py2); 8,59 ppm (1H, dd breit J=4,5 Hz und J=2 Hz,H-Py6); 7,91 ppm (1H,ddd J=8 Hz und J=2 Hz und J=2 Hz,H-Py4); 7,52 ppm (4H,m,H-aromatisch); 7,40 ppm (1H,dd breit J=8 Hz und J=4,5 Hz,H-Py5); 5,88 ppm (1H,s breit,H-4); 5,72 ppm (1H,ddd J=11,5 Hz und J=5,5 Hz und J=5,5 Hz,H-CH=); 5,65 ppm (1H,d breit J=11,5 Hz,H-CH=); 4,28 ppm (2H,m,H-$CH_2OH$); 3,42 ppm (1H,dd breit J=6,0 Hz und J=4,5 Hz,H-11); 2,87 ppm (1H,m,H-10); 0,73 ppm (3H,s,H-18).

**BEISPIEL 8**

Herstellung von 17β-Hydroxy-3-oxo-11β-[4-(3-pyridinyl)phenyl]-4-estren-17α-acetonitril (8B)

A. 3,3-[1,2-Ethandiylbis(thio)]-17β-hydroxy-11β-[4-(3-pyridinyl)phenyl]-4-estren-17α-acetonitril (8A)

Zu einer Lösung von 3,31 ml Diisopropylamin in 100 ml Tetrahydrofuran werden bei -78°C unter Schutzgas 13,7 ml einer 15%igen Lösung von Butyllithium in Hexan getropft. Nach 30 Minuten werden bei der gleichen Temperatur 1,13 ml Acetonitril zugegeben. 15 Minuten später wird bei der gleichen Temperatur eine Lösung von 1,00 g 7F in 90 ml Tetrahydrofuran zugetropft und weitere zwei Stunden gerührt. Dann wird mit gesättigter Ammoniumchloridlösung versetzt und mit Ethylacetat extrahiert. Die vereinigten organischen Phasen werden mit Wasser gewaschen, über Natriumsulfat getrocknet und am Vakuum eingeengt. Säulenchromatographie liefert 1,05 g 8A.

B. 17β-Hydroxy-3-oxo-11β-[4-(3-pyridinyl)phenyl]-4-estren-17α-acetonitril (8B)

Wie unter 3E beschrieben werden aus 1,04 g 8A mit 7,51 g Glyoxylsäure und 6,85 ml 4 M wäßriger Salzsäure in 18 ml Aceton und 18 ml Eisessig 644 mg 8B hergestellt. Durch Kristallisation aus Ethylacetat/Hexan erhält man 597 mg der genannten Verbindung.
Fp.: 173-174°C; $[\alpha]_D^{22}$=+131,8° ($CHCl_3$; c=0,500).
IR (KBr): 2245 $cm^{-1}$ (C≡N), 1664 $cm^{-1}$ (C=O).

**BEISPIEL 9**

Herstellung von (*E*)-17β-Hydroxy-3-(hydroxyimino)-11β-[4-(3-pyridinyl)phenyl]-4-estren-17α-acetonitril (9A) und (*Z*)-17β-Hydroxy-3-(hydroxyimino)-11β-[4-(3-pyridinyl)phenyl]-4-estren-17α-acetonitril (9B)

A. (*E*)-17β-Hydroxy-3-(hydroxyimino)-11β-[4-(3-pyridinyl)phenyl]-4-estren-17α-acetonitril (9A) und
B. (*Z*)-17β-Hydroxy-3-(hydroxyimino)-11β-[4-(3-pyridinyl)phenyl]-4-estren-17α-acetonitril (9B)

219 mg 8B werden mit 50 mg Hydroxylammoniumchlorid in 5 ml Pyridin unter Schutzgas 90 Minuten bei 50°C gerührt. Das Reaktionsgemisch wird in Wasser aufgenommen und mit Methylenchlorid extrahiert. Die organische Phase wird mit Wasser gewaschen, über Natriumsulfat getrocknet und am Vakuum eingeengt. Nach Säulenchromatographie und Kristallisation aus Methylenchlorid/Diisopropylether erhält man 97 mg 9A {Fp.: 227-229°C; $[\alpha]_D^{22}$=+192,4° ($CHCl_3$/MeOH; c=0,500); IR (KBr): 2250 $cm^{-1}$ (C≡N), 1635 $cm^{-1}$ (C=NOH)} sowie 61 mg 9B {Fp.: 198-200°C; $[\alpha]_D^{22}$=+230,4° ($CHCl_3$; c=0,500); IR (KBr): 2250 $cm^{-1}$ (C≡N), 1635 $cm^{-1}$ (C=NOH)}.

**BEISPIEL 10**

Herstellung von 17β-Hydroxy-17α-(2-propenyl)-11β-[4-(3-pyridinyl)phenyl]-4-estren-3-on (10B)

A. 3,3-[1,2-Ethandiylbis(thio)]-17α-(2-propenyl)-11β-[4-(3-pyridinyl)phenyl]-4-estren-17β-ol (10A)

Unter Schutzgas werden 414 mg Magnesiumspäne in 5 ml Diethylether vorgelegt. 150 µl 3-Brom-1-propen und einige Kristalle Iod werden zugegeben, dann wird langsam eine Lösung von 2,00 g 7F und 1,32 g 3-Brom-1-propen in 40 ml Diethylether und 10 ml Tetrahydrofuran zugetropft und drei Stunden am Rückfluß gekocht. Zur Aufarbeitung wird gesättigte Ammoniumchloridlösung zugegeben und mit Ethylacetat extrahiert. Die organische Phase wird mit gesättigter Natriumchloridlösung gewaschen, über Natriumsulfat getrocknet und am Vakuum eingeengt. Säulenchromatographie liefert 1,63 g 10A.

B. 17β-Hydroxy-17α-(2-propenyl)-11β-[4-(3-pyridinyl)phenyl]-4-estren-3-on (10B)

Wie unter 3E beschrieben werden aus 1,44 g 10A mit 10,35 g Glyoxylsäure und 9,40 ml 4 M wäßriger Salzsäure in 25 ml Aceton und 25 ml Eisessig 592 mg 10B hergestellt. Durch Kristallisation aus Ethylacetat / Hexan erhält man 483 mg der genannten Verbindung.
Fp.: 127-128°C; $[\alpha]_D^{22}$=+128,7° ($CHCl_3$; c=0,470).
IR (KBr): 1668 $cm^{-1}$ (C=O).

**BEISPIEL 11**

Herstellung von 17β-Hydroxy-17α-(methoxymethyl)-11β-[4-(3-pyridinyl)phenyl]-4-estren-3-on (11C)

A. (11β,17β)-3,3-[1,2-Ethandiylbis(thio)]-11-[4-(3-pyridinyl)phenyl]spiro[estr-4-en-17,2′-oxiran] (11A)

2,5 g 7F werden mit 4,12 g Trimethylsulfoniumiodid und 2,75 g Kalium-*tert*-butylat in 100 ml Dimethylformamid unter Schutzgas 30 Minuten bei Raumtemperatur gerührt. Dann wird Wasser unter Eisbadkühlung zugegeben und mit Ethylacetat extrahiert. Die organische Phase wird mit gesättigter Natriumchloridlösung gewaschen, über Natriumsulfat getrocknet und am Vakuum eingeengt. Säulenchromatographie liefert 866 mg 11A.

B. 3,3-[1,2-Ethandiylbis(thio)]-17α-(methoxymethyl)-11β-[4-(3-pyridinyl)phenyl]-4-estren-17β-ol (11B)

1,20 g Natrium werden in 15 ml Methanol gelöst. Eine Lösung von 842 mg 11A in 20 ml Methanol wird zugetropft. Das Reaktionsgemisch wird fünf Stunden zum Sieden erhitzt. Dann wird das Methanol weitgehend eingedampft. Der Rückstand wird in Wasser aufgenommen und mit Methylenchlorid extrahiert. Die organische Phase wird mit gesättigter Natriumchloridlösung gewaschen, über Natriumsulfat getrocknet und am Vakuum eingeengt. Säulenchromatographie liefert 486 mg 11B.

C. 17β-Hydroxy-17α-(methoxymethyl)-11β-[4-(3-pyridinyl)phenyl]-4-estren-3-on (11C)

Wie unter 3E beschrieben werden aus 470 mg 11B mit 3,38 g Glyoxylsäure und 3,00 ml 4 M wäßriger Salzsäure in 8 ml Aceton und 8 ml Eisessig 252 mg 11C hergestellt. Durch Kristallisation aus Ethylacetat/Hexan erhält man 204 mg der genannten Verbindung.
Fp.: 145-146°C; $[\alpha]_D^{22}$=+120,8° ($CHCl_3$; c=0,465).
IR (KBr): 1665 $cm^{-1}$ (C=O).

**BEISPIEL 12**

Herstellung von 11β-(4-Ethylphenyl)-17β-hydroxy-17α-(1-propinyl)-4-estren-3-on (12F)

A. 11β-(4-Ethylphenyl)-17β-hydroxy-5(10)-estren-3-on (12A)

Wie unter 4E beschrieben werden aus 863 mg 7B mit 3 ml 4 M wäßriger Salzsäure in 70 ml Aceton 576 mg 12A hergestellt.

B. 11β-(4-Ethylphenyl)-17β-hydroxy-4-estren-3-on (12B)

Wie unter 7D beschrieben werden aus 568 mg 12A mit 500 mg *p*-Toluolsulfonsäure in 10 ml Chloroform 443 mg 12B hergestellt.
IR (KBr): 1665 $cm^{-1}$ (C=O).
$^1$H-NMR($CDCl_3$+Py-$d_5$) δ: 7,31 ppm (2H,d J=9 Hz,H-aromatisch); 7,10 ppm (2H,d J=9 Hz,H-aromatisch); 5,84 ppm (1H,s breit,H-4); 3,59 ppm (1H,dd breit J=9,0 Hz und J=7,5 Hz,H-17); 3,33 ppm (1H,dd breit J=6,0 Hz und J=5,0 Hz,H-11); 2,85 ppm (1H,m,H-10); 2,62 ppm (2H,q J=7,5 Hz,H-Et); 1,23 ppm (2H,t J=7,5 Hz,H-Et); 0,55 ppm (3H,s,H-18).

C. 3,3-[1,2-Ethandiylbis(thio)]-11β-(4-ethylphenyl)-4-estren-17β-ol (12C)

Wie unter 4G beschrieben werden aus 433 mg 12B mit 85 µl 1,2-Ethandithiol und 285 mg *p*-Toluolsulfonsäure in 5 ml Eisessig sowie mit 140 mg Kaliumcarbonat in 6 ml Methylenchlorid und 30 ml Methanol 399 mg 12C hergestellt.

D. 3,3-[1,2-Ethandiylbis(thio)]-11β-(4-ethylphenyl)-4-estren-17-on (12D)

Wie unter 3C beschrieben werden aus 390 mg 12C mit 360 µl Cyclohexanon und 70 mg Aluminiumtriisopropylat in 15 ml Toluol 347 mg 12D hergestellt.
IR (KBr): 1740 $cm^{-1}$ (C=O).

E. 3,3-[1,2-Ethandiylbis(thio)]-11β-(4-ethylphenyl)-17α-(1-propinyl)-4-estren-17β-ol (12E)

Wie unter 1L beschrieben werden aus 100 mg 12D mit 800 µl einer 15%igen Lösung von Butyllithium in Hexan in 10 ml mit Propin gesättigtem Tetrahydrofuran 99 mg 12E hergestellt.
IR (KBr): 2235 $cm^{-1}$ (C≡C).

F. 11β-(4-Ethylphenyl)-17β-hydroxy-17α-(1-propinyl)-4-estren-3-on (12F)

Wie unter 3E beschrieben werden aus 89 mg 12E mit 64 mg Glyoxylsäure und 600 µl 4 M wäßriger Salzsäure in 2 ml Aceton und 2 ml Eisessig nach Kristallisation aus Methylenchlorid / Hexan 34 mg 12F hergestellt.
Fp.: 195-196°C; $[\alpha]_D^{22}$=-17,9° ($CHCl_3$; c=0,485).
IR (KBr): 2240 $cm^{-1}$ (C≡C), 1655 $cm^{-1}$ (C=O).

**BEISPIEL 13**

Herstellung von (*Z*)-11β-(4-Ethylphenyl)-17β-hydroxy-17α-(3-hydroxy-1-propenyl)-4-estren-3-on (13C)

A. 3,3-[1,2-Ethandiylbis(thio)]-11β-(4-ethylphenyl)-17α-[3-[(tetrahydro-2*H*-pyran-2-yl)oxy]-1-propinyl]-4-estren-17β-ol (13A)

Wie unter 3D beschrieben werden aus 235 mg 12D mit 0,70 ml 2-(2-Propinyloxy)tetrahydro-2*H*-pyran und 2,90 ml einer 15%igen Lösung von Butyllithium in Hexan in 40 ml Tetrahydrofuran 241 mg 13A hergestellt.

B. 11β-(4-Ethylphenyl)-17β-hydroxy-17α-(3-hydroxy-1-propinyl)-4-estren-3-on (13B)

Wie unter 3E beschrieben werden aus 230 mg 13A mit 1,60 g Glyoxylsäure und 1,50 ml 4 M wäßriger Salzsäure in 5 ml Aceton und 5 ml Eisessig 124 mg 13B hergestellt.
IR (KBr): 2230 $cm^{-1}$ (C≡C), 1660 $cm^{-1}$ (C=O).

C. (Z)-11β-(4-Ethylphenyl)-17β-hydroxy-17α-(3-hydroxy-1-propenyl)-4-estren-3-on (13C)

Wie unter 3F beschrieben werden 115 mg 13B mit 25 mg 10%igem Pd/$BaSO_4$-Katalysator in 1 ml Pyridin und 4 ml Ethanol hydriert. Nach Kieselgelchromatographie und Kristallisation aus Methylenchlorid / Hexan werden 76 mg der Titelverbindung 13C erhalten.
Fp.: 98-101°C; $[\alpha]_D^{22}$=+76,6° ($CHCl_3$; c=0,500).
IR(KBr): 1665 $cm^{-1}$ (C=O).

**BEISPIEL 14**

Herstellung von (*Z*)-11β-[4-(3-Furanyl)phenyl]-17β-hydroxy-17α-(3-hydroxy-1-propenyl)-4-estren-3-on (14D)

A. 3,3-[2,2-Dimethyl-1,3-propandiylbis(oxy)]-11β-[4-(3-furanyl)phenyl]-5(10)-estren-17β-ol (14A)

Unter Schutzgas werden zu einer Lösung von 3,50 g 4C in 25 ml Dioxan und 0,46 ml Pyridin 6,43 g (3-Furanyl)tributylstannan (Herstellung siehe Synthesis **1985**, 898), 510 mg Lithiumchlorid, und 693 mg Tetrakis(triphenylphosphin)palladium(0) gegeben. Das Gemisch wird zwei Stunden zum Sieden erhitzt, nach dem Abkühlen in Ethylacetat aufgenommen und über Celite filtriert. Die organische Phase wird über Natriumsulfat getrocknet und im Vakuum eingeengt. Nach Säulenchromatographie werden 2,79 g 14A erhalten.

B. 11β-[4-(3-Furanyl)phenyl]-17β-hydroxy-4-estren-3-on (14B)

Aus 2,70 g 14A werden analog den Vorschriften 4E und 7D durch Ketalhydrolyse und Isomerisierung 1,03 g der Verbindung 14B hergestellt.
$^1$H-NMR($CDCl_3$) δ: 7,74 ppm (1H, dd J=1,3 Hz und J=0,8 Hz,H-Fu2); 7,48 ppm (1H,dd J=1,8 Hz und J=1,3 Hz,H-Fu5); 7,42 ppm (4H,m,H-aromatisch); 6,71 ppm (1H,dd J=1,8 Hz und J=0,8 Hz,H-Fu4); 5,87 ppm (1H,s breit,H-4); 3,60 ppm (1H,dd J=9,0 Hz und J=7,0 Hz,H-17); 3,36 ppm (1H,dd breit J=6,0 Hz und J=5,0 Hz,H-11); 2,85 ppm (1H,m,H-10); 0,57 ppm (3H,s,H-18).

C. 11β-[4-(3-Furanyl)phenyl]-17β-hydroxy-17α-(3-hydroxy-1-propinyl)-4-estren-3-on (14C)

Aus 1,00 g 14B werden analog den Vorschriften 4G, 3C, 3D und 3E 358 mg der Verbindung 14C hergestellt.
IR (KBr): 2230 $cm^{-1}$ (C≡C), 1660 $cm^{-1}$ (C=O).

D. (Z)-11β-[4-(3-Furanyl)phenyl]-17β-hydroxy-17α-(3-hydroxy-1-propenyl)-4-estren-3-on (14D)

Wie unter 3F beschrieben werden 344 mg 14C mit 70 mg 10%igem Pd/$BaSO_4$-Katalysator in 3 ml Pyridin und 6 ml Ethanol hydriert. Nach Kieselgelchromatographie und Kristallisation aus Ethylacetat / Hexan werden 180 mg der Titelverbindung 14D erhalten.
Fp.: 164-166°C; $[\alpha]_D^{22}$=+99,0° ($CHCl_3$; c=0,200).
IR (KBr): 1662 $cm^{-1}$ (C=O).

**BEISPIEL 15**

Herstellung von (*Z*)-11β-[4-(2-Furanyl)phenyl]-17β-hydroxy-17α-(3-hydroxy-1-propenyl)-4-estren-3-on (15D)

A. 3,3-[2,2-Dimethyl-1,3-propandiylbis(oxy)]-11β-[4-(2-furanyl)phenyl]-5(10)-estren-17β-ol (15A)

Unter Schutzgas werden zu einer Lösung von 4,00 g 4C in 65 ml Toluol und 25 ml Ethanol 1,52 g (2-Furanyl)boronsäure (Herstellung siehe J. Heterocycl. Chem. **1975**, 195), 580 mg Lithiumchlorid, 8,5 ml 2 M Natriumcarbonatlösung und 340 mg Tetrakis(triphenylphosphin)palladium(0) gegeben. Das Gemisch wird vier Stunden zum Sieden erhitzt und nach dem Abkühlen in Ethylacetat aufgenommen. Die organische Phase wird mit 1 M Natronlauge sowie mit gesättigter Natriumchloridlösung gewaschen, über Natriumsulfat getrocknet und im Vakuum eingeengt. Nach Säulenchromatographie werden 2,57 g 15A erhalten.

B. 11β-[4-(2-Furanyl)phenyl]-17β-hydroxy-4-estren-3-on (15B)

Aus 2,52 g 15A werden analog den Vorschriften 4E und 7D durch Ketalhydrolyse und Isomerisierung 1,10 g der Verbindung 15B hergestellt.
IR (KBr): 1664 $cm^{-1}$ (C=O).
$^1$H-NMR($CDCl_3$+Py-$d_5$) δ: 7,60 ppm (2H,d J=9 Hz,H-aromatisch); 7,47 ppm (1H, dd J=1.8 Hz und J=0,8 Hz,H-Fu5); 7,44 ppm (2H,d J=9 Hz,H-aromatisch); 6,62 ppm (1H,dd J=3,5 Hz und J=1,8 Hz,H-Fu3); 6,48 ppm (1H,dd J=3,5 Hz und J=0,8 Hz,H-Fu4); 5,85 ppm (1H,s breit,H-4); 3,61 ppm (1H,dd J=9,0 Hz und J=7,0 Hz,H-17); 3,38 ppm (1H,dd breit J=5,5 Hz und J=5,0 Hz,H-11); 2,84 ppm (1H,m,H-10); 0,56 ppm (3H,s,H-18).

C. 11β-[4-(2-Furanyl)phenyl]-17β-hydroxy-17α-(3-hydroxy-1-propinyl)-4-estren-3-on (15C)

Aus 1,07 g 15B werden analog den Vorschriften 4G, 3C, 3D und 3E 313 mg der Verbindung 15C hergestellt.
IR (KBr): 2235 $cm^{-1}$ (C≡C), 1665 $cm^{-1}$ (C=O).

D. (*Z*)-11β-[4-(2-Furanyl)phenyl]-17β-hydroxy-17α-(3-hydroxy-1-propenyl)-4-estren-3-on (15D)

Wie unter 3F beschrieben werden 299 mg 15C mit 61 mg 10%igem Pd/$BaSO_4$-Katalysator in 3 ml Pyridin und 5 ml Ethanol hydriert. Nach Kieselgelchromatographie und Kristallisation aus Ethylacetat / Hexan werden 205 mg der Titelverbindung 15D erhalten.
Fp.: 148-149°C; $[\alpha]_D^{22}$=+126,5° ($CHCl_3$; c=0,350).
IR (KBr): 1660 $cm^{-1}$ (C=O).

**BEISPIEL 16**

Herstellung von (*Z*)-4′-[17β-Hydroxy-17α-(3-hydroxy-1-propenyl)-3-oxo-4-estren-11β-yl][1,1′-biphenyl]-4-carbonitril (16D)

A. 4′-[3,3-[2,2-Dimethyl-1,3-propandiylbis(oxy)]-17β-hydroxy-5(10)-estren-11β-yl][1,1′-biphenyl]-4-carbonitril (16A)

Eine Lösung von 5,00 g 4C in 170 ml Dioxan wird mit 1,09 g Lithiumchlorid 15 Minuten bei Raumtemperatur unter Schutzgas gerührt. 13 ml Hexabutyldizinn und 494 mg Tetrakis(triphenylphosphin)palladium(0) werden zugegeben. Das Reaktionsgemisch wird zwei Stunden zum Sieden erhitzt. Dann werden 15,6 g 4-Brombenzonitril zugefügt und der Ansatz wird weitere 24 Stunden zum Sieden erhitzt. Nach dem Erkalten wird über Celite filtriert, mit Ethylacetat nachgewaschen und am Vakuum eingeengt. Nach Säulenchromatographie erhält man 3,16 g 16A.

B. 4′-[17β-Hydroxy-3-oxo-4-estren-11β-yl][1,1′-biphenyl]-4-carbonitril (16B)

Aus 3,10 g 16A werden analog den Vorschriften 4E und 7D durch Ketalhydrolyse und Isomerisierung 1,36 g der Verbindung 16B hergestellt.
IR (KBr): 2233 $cm^{-1}$ (C≡N); 1660 $cm^{-1}$ (C=O).
$^1H$-NMR($CDCl_3$+Py-$d_5$) δ: 7,73 ppm (2H,d J=9 Hz,H-aromatisch); 7,70 ppm (2H,d J=9 Hz,H-aromatisch); 7,53 ppm (4H,m,H-aromatisch); 5,87 ppm (1H,s breit,H-4); 3,60 ppm (1H,dd J=9,0 Hz und J=7,5 Hz,H-17); 3,43 ppm (1H,dd breit J=6,0 Hz und J=5,0 Hz,H-11); 2,85 ppm (1H,m,H-10); 0,57 ppm (3H,s,H-18).

C. 4′-[17β-Hydroxy-17α-(3-hydroxy-1-propinyl)-3-oxo-4-estren-11β-yl][1,1′-biphenyl]-4-carbonitril (16C)

Aus 1,32 g 16B werden analog den Vorschriften 4G, 3C, 3D und 3E 405 mg der Verbindung 16C hergestellt.
IR (KBr): 2230 $cm^{-1}$ (C≡N und C≡C); 1658 $cm^{-1}$ (C=O).

D. (*Z*)-4′-[17β-Hydroxy-17α-(3-hydroxy-1-propenyl)-3-oxo-4-estren-11β-yl][1,1′-biphenyl]-4-carbonitril (16D)

Wie unter 3F beschrieben werden 386 mg 16C mit 77 mg 10%igem Pd/$BaSO_4$-Katalysator in 4 ml Pyridin und 6 ml Ethanol hydriert. Nach Kieselgelchromatographie und Kristallisation aus Ethylacetat / Hexan werden 242 mg der Titelverbindung 16D erhalten.
Fp.: 259-260°C; $[\alpha]_D^{22}$=+135,2° ($CHCl_3$; c=0,510).
IR (KBr): 2230 $cm^{-1}$ (C≡N); 1664 $cm^{-1}$ (C=O).

**BEISPIEL 17**

Herstellung von 11β-(4-Ethenylphenyl)-17β-hydroxy-17α-methyl-4-estren-3-on (17E)

A. 3,3-[2,2-Dimethyl-1,3-propandiylbis(oxy)]-11β-(4-ethenylphenyl)-5(10)-estren-17β-ol (17A)

Unter Schutzgas werden zu einer Lösung von 4,00 g 4C in 50 ml Dimethylformamid 2,40 ml Ethenyltributylstannan, 554 mg Lithiumchlorid, und 378 mg Tetrakis(triphenylphosphin)palladium(0) gegeben. Das Gemisch wird 150 Minuten auf 110°C erhitzt, nach dem Abkühlen in Ethylacetat aufgenommen und über Celite filtriert. Das Filtrat wird mit gesättigter Natriumchloridlösung gewaschen, über Natriumsulfat getrocknet und im Vakuum eingeengt. Nach Säulenchromatographie werden 3,11 g 17A erhalten.

B. 11β-(4-Ethenylphenyl)-17β-hydroxy-4-estren-3-on (17B)

Aus 3,08 g 17A werden analog den Vorschriften 4E und 7D durch Ketalhydrolyse und Isomerisierung 1,09 g der Verbindung 17B hergestellt.
IR (KBr): 1663 $cm^{-1}$ (C=O).

C. 3,3-[1,2-Ethandiylbis(thio)]-11β-(4-ethenylphenyl)-4-estren-17-on (17C)

Aus 1,06 g 17B werden analog den Vorschriften 4G und 3C durch Thioketalisierung und Oxidation 805 mg der Verbindung 17C hergestellt.
IR (KBr): 1736 $cm^{-1}$ (C=O).

D. 3,3-[1,2-Ethandiylbis(thio)]-11β-(4-ethenylphenyl)-17α-methyl-4-estren-17β-ol (17D)

Bei 0°C werden zu einer Lösung von 780 mg 17C in 20 ml Tetrahydrofuran unter Schutzgas 11,9 ml einer 1,6 M Lösung von Methyllithium in Diethylether getropft. Die Reaktionsmischung wird 30 Minuten nachgerührt und dann in gesättigte Ammoniumchloridlösung gegossen. Die wäßrige Phase wird mit Ethylacetat extrahiert. Die organische Phase wird mit Wasser gewaschen, über Natriumsulfat getrocknet und am Vakuum eingeengt. Nach Säulenchromatographie werden 629 mg 17D erhalten.

E. 11β-(4-Ethenylphenyl)-17β-hydroxy-17α-methyl-4-estren-3-on (17E)

Wie unter 3E beschrieben werden aus 605 mg 17D mit 4,23 g Glyoxylsäure und 3,70 ml 4 M wäßriger Salzsäure in 10 ml Aceton und 10 ml Eisessig 216 mg der Titelverbindung 17E hergestellt.
$[\alpha]_D^{22}$=+151,8° ($CHCl_3$; c=0,505).
IR (KBr): 1666 $cm^{-1}$ (C=O).
$^1$H-NMR($CDCl_3$) δ: 7,39 ppm (2H,d J=9,0 Hz,H-aromatisch); 7,33 ppm (2H,d J=9,0 Hz,H-aromatisch); 6,70 ppm (1H,dd J-17,0 Hz und J-10,5 Hz,H-Vinyl); 5,86 ppm (1H,s breit,H-4); 5,73 ppm (1H,d J=17,0 Hz,H-Vinyl); 5,22 ppm (1H,d J=10,5 Hz,H-Vinyl); 3,39 ppm (1H,dd breit J=9,5 Hz und J=4,5 Hz,H-11); 2,87 ppm (1H,m,H-10); 1,22 ppm (3H,s,H-$CH_3$); 0,66 ppm (3H,s,H-18).

**BEISPIEL 18**

Herstellung von (*Z*)-17β-Hydroxy-17α-(3-hydroxy-1-propenyl)-11β-(4-methnylphenyl)-4-estren-3-on (18D)

A. 3,3-[2,2-Dimethyl-1,3-propandiylbis(oxy)]-11β-(4-methylphenyl)-5(10)-estren-17β-ol (18A)

Zu einer Suspension von 2,10 g Kupfer(I)cyanid in 40 ml Diethylether und 10 ml Tetrahydrofuran werden bei -78°C unter Schutzgas 27,8 ml einer 1,6 M Lösung von Methyllithium in Diethylether getropft. Die Reaktionslösung wird auf -20°C erwärmt und nach 15 Minuten wiederum auf -78°C abgekühlt. Bei dieser Temperatur wird eine Lösung von 4,00 g 4C in 25 ml Diethylether zugetropft. Das Reaktionsgemisch wird langsam auf -20°C erwärmt und bei dieser Temperatur zwei Tage gerührt. Zur Aufarbeitung wird gesättigte Ammoniumchloridlösung zugesetzt und mit Ethylacetat extrahiert. Die organische Phase wird mit Wasser und mit gesättigter Natriumchloridlösung gewaschen, über Natriumsulfat getrocknet und am Vakuum eingeengt. Säulenchromatographie liefert 1,65 g 18A.

B. 17β-Hydroxy-11β-(4-methylphenyl)-4-estren-3-on (18B)

Aus 1,61 g 18A werden analog den Vorschriften 4E und 7D durch Ketalhydrolyse und Isomerisierung 920 mg der Verbindung 18B hergestellt.
IR (KBr): 1662 $cm^{-1}$ (C=O).

C. 17β-Hydroxy-17α-(3-hydroxy-1-propinyl)-11β-(4-methylphenyl)-4-estren-3-on (18C)

Aus 895 mg 18B werden analog den Vorschriften 4G, 3C, 3D und 3E 294 mg der Verbindung 18C hergestellt.
$[\alpha]_D^{22}$=-26,6° ($CHCl_3$; c=0,500).
IR (KBr): 2230 $cm^{-1}$ (C≡C), 1659 $cm^{-1}$ (C=O).
$^1$H-NMR($CDCl_3$) δ: 7,29 ppm (2H,d J=8.0 Hz,H-aromatisch); 7,09 ppm (2H,d J=8,0 Hz,H-aromatisch); 5,86 ppm (1H,s breit,H-4); 4,37 ppm (2H,m,H-$CH_2OH$); 3,39 ppm (1H,dd breit J=6,5 Hz und J=5,0 Hz,H-11); 2,85 ppm (1H,m,H-10); 2,32 ppm (3H,s,H-$CH_3$); 0,76 ppm (3H,s,H-18).

D. (Z)-17β-Hydroxy-17α-(3-hydroxy-1-propenyl)-11β-(4-methylphenyl)-4-estren-3-on (18D)

Wie unter 3F beschrieben werden 265 mg 18C mit 53 mg 10%igem Pd/$BaSO_4$-Katalysator in 3 ml Pyridin und 6 ml Ethanol hydriert. Nach Kieselgelchromatographie werden 193 mg der Titelverbindung 18D erhalten.
$[\alpha]_D^{22}$=+75,8° ($CHCl_3$; c=0,510).
IR (KBr): 1664 $cm^{-1}$ (C=O).
$^1$H-NMR($CDCl_3$) δ: 7,29 ppm (2H,d J=8,0 Hz,H-aromatisch); 7,09 ppm (2H,d J=8,0 Hz,H-aromatisch); 5,85 ppm (1H,s breit,H-4); 5,71 ppm (1H,ddd J=12,0 Hz und J=5,5 Hz und J=5,5 Hz,H-CH=); 5,62 ppm (1H,d breit J=12,0 Hz,H-CH=); 4,24 ppm (2H,m,H-$CH_2$OH); 3,33 ppm (1H,dd breit J=6,0 Hz und J=5,0 Hz,H-11); 2,84 ppm (1H,m,H-10); 2,32 ppm (3H,s,H-$CH_3$); 0,71 ppm (3H,s,H-18).

Herstellung von (Z)-17β-Hydroxy-17α-(3-hydroxy-1-propenyl)-11β-[4-(5-pyrimidinyl)phenyl]-4-estren-3-on (19D)

A. 3,3-[2,2-Dimethyl-1,3-propandiylbis(oxy)]-11β-[4-(5-pyrimidinyl)phenyl]-5(10)-estren-17β-ol (19A)

Eine Lösung von 4,40 g 4C in 300 ml Dioxan wird mit 1,35 g Lithiumchlorid 15 Minuten bei Raumtemperatur unter Schutzgas gerührt. 8,3 ml Hexabutyldizinn und 735 mg Tetrakis(triphenylphosphin)palladium(0) werden zugegeben. Das Reaktionsgemisch wird 90 Minuten auf 100°C erhitzt. Dann werden 2,3 g 5-Brompyrimidin zugefügt und der Ansatz wird weitere 7 Stunden zum Sieden erhitzt. Nach dem Erkalten wird über Celite filtriert, mit Ethylacetat nachgewaschen und am Vakuum eingeengt. Nach Säulenchromatographie erhält man 2,37 g 19A.

B. 17β-Hydroxy-11β-11β-[4-(5-pyrimidinyl)phenyl]-4-estren-3-on (19B)

Aus 2,31 g 19A werden analog den Vorschriften 4E und 7D durch Ketalhydrolyse und Isomerisierung 991 mg der Verbindung 19B hergestellt.
IR (KBr): 1660 $cm^{-1}$ (C=O).
$^1$H-NMR($CDCl_3$+Py-$d_5$) δ: 9,19 ppm (1H,s breit,H-Py2); 8,99 ppm (2H,s breit,H-Py4 und Py6); 7,60 ppm (2H,d J=9 Hz,H-aromatisch); 7,53 ppm (2H,d J=9 Hz,H-aromatisch); 5,87 ppm (1H,s breit,H-4); 3,60 ppm (1H,dd J=8,5 Hz und J=7,0 Hz,H-17); 3,43 ppm (1H,dd breit J=6,0 Hz und J=5,0 Hz,H-11); 2,84 ppm (1H,m,H-10); 0,57 ppm (3H,s,H-18).

C. 17β-Hydroxy-17α-(3-hydroxy-1-propinyl)-11β-[4-(5-pyrimidinyl)phenyl]-4-estren-3-on (19C)

Aus 955 mg 19B werden analog den Vorschriften 4G, 3C, 3D und 3E 203 mg der Verbindung 19C hergestellt.
IR (KBr): 2235 $cm^{-1}$ (C≡C), 1665 $cm^{-1}$ (C=O).

D. (Z)-17β-Hydroxy-17α-(3-hydroxy-1-propenyl)-11β-[4-(5-pyrimidinyl)phenyl]-4-estren-3-on (19D)

Wie unter 3F beschrieben werden 187 mg 19C mit 37 mg 10%igem Pd/$BaSO_4$-Katalysator in 2 ml Pyridin und 4 ml Ethanol hydriert. Nach Kieselgelchromatographie werden 112 mg der Titelverbindung 19D erhalten.
IR (KBr): 1664 $cm^{-1}$ (C=O).
$^1$H-NMR($CDCl_3$) δ: 9,20 ppm (1H,s breit,H-Py2); 8,98 ppm (2H,s breit,H-Py4 und Py6); 7,59 ppm (2H,d J=9 Hz,H-aromatisch); 7,52 ppm (2H,d J=9 Hz,H-aromatisch); 5,88 ppm (1H,s breit,H-4); 5,72 ppm (1H,ddd J=11,5 Hz und J=5,5 Hz und J=5,5 Hz,H-CH=); 5,65 ppm (1H,d breit J=11,5 Hz,H-CH=); 4,29 ppm (1H,dd breit J=14 Hz und J=5,5 Hz,H-$CH_2$OH); 4,26 ppm (1H,dd breit J=14 Hz und J=5,5 Hz,H-$CH_2$OH); 3,44 ppm (1H,dd breit J=6,0 Hz und J=5,0 Hz,H-11); 2,85 ppm (1H,m,H-10); 0,72 ppm (3H,s,H-18).

**BEISPIEL 20**

Herstellung von (11β,17β)-11-[4-(5-Pyrimidinyl)phenyl]spiro[estr-4-en-17,2′(3′H)-furan]-3-on (20A)

A. (11β,17β)-11-[4-(5-Pyrimidinyl)phenyl]spiro[estr-4-en-17,2′(3′H)-furan]-3-on (20A)

320 mg der unter 19D beschriebenen Verbindung werden bei 0°C mit 400 mg p-Toluolsulfonsäurechlorid und 1 ml Triethylamin zusammengegeben. Das Reaktionsgemisch wird zwölf Stunden bei Raumtemperatur gerührt und dann mit gesättigter Natriumhydrogencarbonatlösung versetzt und mit Methylenchlorid extrahiert. Die organische Phase wird mit gesättigter Natriumchloridlösung gewaschen, über Natriumsulfat getrocknet und am Vakuum eingeengt. Säulenchromatographie liefert 140 mg 20A.
$^1$H-NMR($CDCl_3$) δ: 9,20 ppm (1H,s breit,H-Py2); 8,98 ppm (2H,s breit,H-Py4 und Py6); 7,57 ppm (2H,d J=9 Hz,H-aromatisch); 7,52 ppm (2H,d J=9 Hz,H-aromatisch); 5,88 ppm (1H,s breit,H-4); 5,86 ppm (1H,m,H-CH=); 5,81 ppm (1H,m,H-CH=); 4,52 ppm (1H,d breit J=14 Hz,H-$CH_2OC$); 4,49 ppm (1H,d breit J=14 Hz,H-$CH_2OC$); 3,39 ppm (1H,dd breit J=9,0 Hz und J=4,0 Hz,H-11); 2,84 ppm (1H,m,H-10); 0,71 ppm (3H,s,H-18).

## Patentansprüche

1. 11β-Aryl-4-estrene der allgemeinen Formel I

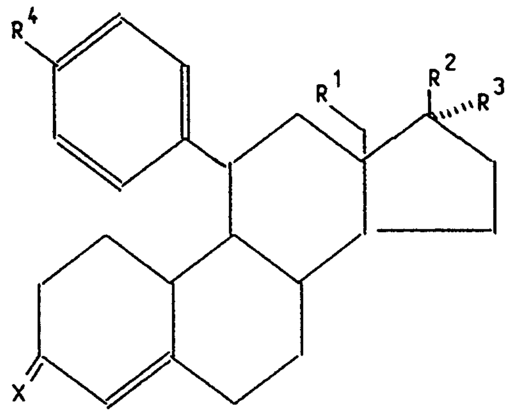

(I),

worin

X für ein Sauerstoffatom, die Hydroxyiminogruppierung > N~OH oder zwei Wasserstoffatome,
$R^1$ für ein Wasserstoffatom oder eine Methylgruppe,
$R^2$ für eine Hydroxygruppe, eine $C_1$-$C_{10}$-Alkoxy- oder $C_1$-$C_{10}$-Acyloxygruppe,
$R^3$ für ein Wasserstoffatom, die Gruppierung $-(CH_2)_nCH_2Z$, wobei n 0, 1, 2, 3, 4 oder 5 ist, Z ein Wasserstoffatom, die Cyanogruppe oder den Rest - $OR^5$ mit $R^5$=H, $C_1$-$C_{10}$-Alkyl oder $C_1$-$C_{10}$-Acyl bedeuten, die Gruppierung $-(CH_2)_mC{\equiv}C-Y$, wobei m 0, 1 oder 2 und Y ein Wasserstoff-, Fluor-, Chlor- Brom-oder Jod-Atom, einen $C_1$-$C_{10}$-Hydroxyalkyl-, $C_1$-$C_{10}$-Alkoxyalkyl-, $C_1$-$C_{10}$-Acyloxyalkylrest bedeuten, die Gruppierung $-(CH_2)_p-CH{=}CH-(CH_2)_kCH_2R^6$, wobei p 0 oder 1 und k 0, 1 oder 2 und $R^6$ ein Wasserstoffatom, eine Hydroxygruppe, einen $C_1$-$C_4$-Alkoxy- oder $C_1$-$C_4$-Acyloxyrest bedeuten,
oder aber $R^2$ und $R^3$ gemeinsam für einen Rest der Formel

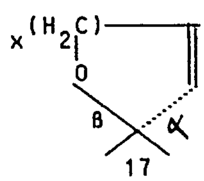

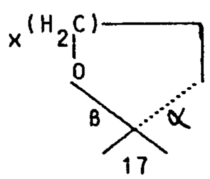

oder

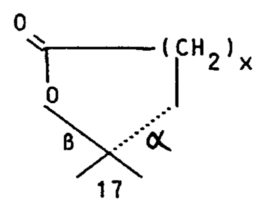

wobei x = 1 oder 2 ist $R^4$
für ein Wasserstoffatom, eine Cyanogruppe, ein Chlor-, Fluor-, Brom-, Jodatom, für eine Trialkylsilyl-, Trialkylstannylgruppe, für einen geradkettigen oder verzweigten, gesättigten oder ungesättigten $C_1$-$C_8$-Alkyl-, -Acyl- oder Alkoxyalkylrest, für eine Aminogruppe

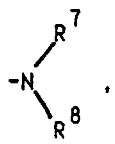

,

in welcher $R^7$ und $R^8$ unabhängig voneinander ein Wasserstoffatom oder eine $C_1$-$C_4$-Alkylgruppe bedeuten, oder

für ein entsprechendes Aminoxid

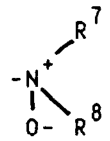 ,

oder für die Gruppierungen -OR⁹ oder -S(O)ᵢR⁹ mit i = 0, 1 oder 2, in welchen R⁹ ein Wasserstoffatom, eine Methyl-, Ethyl-, Propyl-, Isopropyl-, Methoxyphenyl-, Allyl-oder eine 2-Dimethylaminoethylgruppe bedeuten, oder für einen Heteroarylrest der Formel Iα

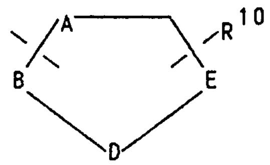 (Iα),

in welchem A ein Stickstoff-, Sauerstoff- oder Schwefelatom, -B-D-E- die Elementenfolge -C-C-C-, -N-C-C- oder -C-N-C- und $R^{10}$ ein Wasserstoffatom, eine Cyanogruppe, ein Chlor-, Fluor-, Brom-, Jodatom, eine Trialkylsilyl-, Trialkylstannylgruppe, einen geradkettigen oder verzweigten, gesättigten oder ungesättigten $C_1$-$C_8$-Alkyl-, -Acyl- oder Alkoxyalkylrest, für eine Aminogruppe

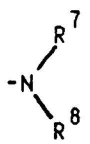 ,

in welcher $R^7$ und $R^8$ unabhängig voneinander ein Wasserstoffatom oder eine $C_1$-$C_4$-Alkylgruppe bedeuten, oder ein entsprechendes Aminoxid

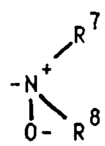 ,

oder die Gruppierung $-OR^9$ oder $-S(O)_iR^9$ mit i = 0, 1 oder 2, in welchen $R^9$ ein Wasserstoffatom, eine Methyl-, Ethyl-, Propyl-, Isopropyl-, Methoxyphenyl-, Allyl-oder eine 2-Dimethylaminoethylgruppe bedeuten, symbolisieren,
oder für einen Heteroarylrest der Formel Iβ

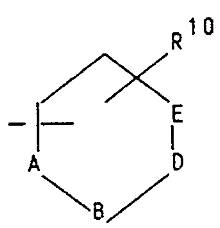 (Iβ),

in welchem A ein Stickstoffatom und -B-D-E- die Elementenfolge -C-C-C-, -N-C-C-, -C-N-C- oder -C-C-N- bedeuten und $R^{10}$ die bereits angegebene Bedeutung hat,
oder für einen Phenylrest der Formel Iγ

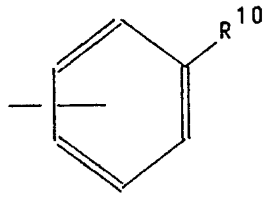

(Iγ),

worin $R^{10}$ die bereits angegebene Bedeutung hat,
stehen,
sowie deren pharmakologisch verträgliche Additionssalze mit Säuren.

**2.** 11β-[4-(Dimethylamino)phenyl]-17β-hydroxy-17α-(1-propinyl)-4-estren-3-on;
11β-(4-Acetylphenyl)-17β-hydroxy-4-estren-3-on;
11β-(4-Acetylphenyl)-17β-hydroxy-17α-(1-propinyl)-4-estren-3-on;
11β-(4-Acetylphenyl)-17β-hydroxy-17α-(3-hydroxy-1Z-propenyl)-4-estren-3-on;
11β-4-(Acetylphenyl)-17β-hydroxy-17α-(3-hydroxy1Z-propenyl)-4-estren-3-on;
11β-[4-(Dimethylamino)phenyl]-17β-hydroxy-17α-(3-hydroxy-1Z-propenyl)-4-estren-3-on;
11β-[4-(3-Furyl)phenyl]-17β-hydroxy-17α-(1-propinyl)-4-estren-3-on;
11β-[4-(3-Furyl)phenyl]-17β-hydroxy-17α-(3-hydroxy-1Z-propenyl)-4-estren-3-on;
11β-[4-(5-Pyrimidinyl)phenyl]-17β-hydroxy-17α-(1-propinyl)-4-estren-3-on;
11β-[4-(5-Pyrimidinyl)phenyl]-17β-hydroxy-17α-(3-hydroxy-1Z-propenyl)-4-estren-3-on;
11β-[4-(3-Pyridyl)phenyl]-17β-hydroxy-17α-(1-propinyl)-4-estren-3-on;
11β-[4-(3-Pyridyl)phenyl]-17β-hydroxy-17α-(3-hydroxy-1Z-propenyl)-4-estren-3-on;
11β-[4-(4-Cyanophenyl)phenyl]-17β-hydroxy-17α-(1-propinyl)-4-estren-3-on;
11β-[4-(4-Cyanophenyl)phenyl]-17β-hydroxy-17α-(3-hydroxy-1Z-propenyl)-4-estren-3-on;
11β-(4-Vinylphenyl)-17β-hydroxy-17α-(3-hydroxy-1Z-propenyl)-4-estren-3-on;
11β-(4-Vinylphenyl)-17β-hydroxy-17α-(1-propinyl)-4-estren-3-on;
11β-[4-(1-Hydroxyethyl)phenyl]-17β-hydroxy-17α-(3-hydroxy-1Z-propenyl)-4 estren-3-on;
11β-[4-(Dimethylamino)phenyl]-17β-hydroxy-17α-methoxymethyl)-4-estren-3-on;
11β-[4-(Dimethylamino)phenyl]-17β-hydroxy-17α-cyanomethyl)-4-estren-3-on;
(11β,17β)-4',5'-Dihydro-11-[4-(dimethylamino)phenyl]spiro[estr-4-en-17,2'-(3'H)-furan]-3-on
(11β,17β)-3',4'-Dihydro-11-[4-(dimethylamino)phenyl]spiro[estr-4-en-17,2'-(3'H)-furan]-3,5'-dion
(11β,17β)-11-[4-(Dimethylamino)phenyl]spiro[estr-4-en-17,2'(5'H)-furan]-3-on
11β-[4-(Dimethylamino)phenyl]-17α-(1-propinyl)-4-estren-17β-ol
17β-Hydroxy-3-oxo-11β-[4-(3-pyridinyl)phenyl]-4-estren-17α-acetonitril
(*E*)-17β-Hydroxy-3-(hydroxyimino)-11β-[4-(3-pyridinyl)phenyl]-4-estren-17α-acetonitril
(*Z*)-17β-Hydroxy-3-(hydroxyimino)-11β-[4-(3-pyridinyl)phenyl]-4-estren-17α-acetonitril
17β-Hydroxy-17α-(2-propenyl)-11β-[4-(3-pyridinyl)phenyl]-4-estren-3-on
17β-Hydroxy-17α-(methoxymethyl)-11β-[4-(3-pyridinyl)phenyl]-4-estren-3-on
11β-(4-Ethylphenyl)-17β-hydroxy-17α-(1-propinyl)-4-estren-3-on
(*Z*)-11β-(4-Ethylphenyl)-17β-hydroxy-17α-(3-hydroxy-1-propenyl)-4-estren-3-on
(*Z*)-11β-[4-(2-Furanyl)phenyl]-17β-hydroxy-17α-(3-hydroxy-1-propenyl)-4-estren-3-on
11β-(4-Ethenylphenyl)-17β-hydroxy-17α-methyl-4-estren-3-on
(*Z*)-17β-Hydroxy-17α-(3-hydroxy-1-propenyl)-11β-(4-methylphenyl)-4-estren-3-on
(11β,17β)-11-[4-(5-Pyrimidinyl)phenyl]spiro[estr-4-en-17,2'(3'H)-furan]-3-on .

3. Verfahren zur Herstellung von 11β-Aryl-4-estrenen der allgemeinen Formel I

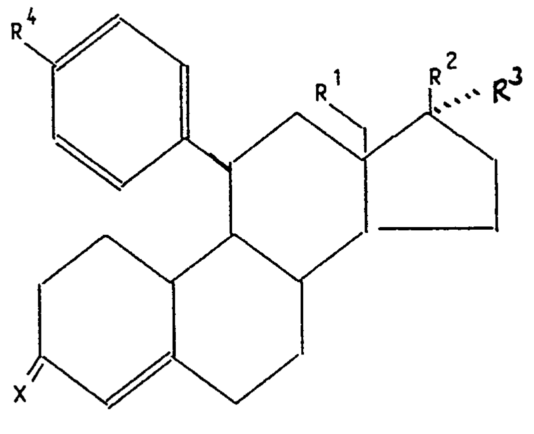

(I),

worin

X für ein Sauerstoffatom, die Hydroxyiminogruppierung >N~OH oder zwei Wasserstoffatome,
$R^1$ für ein Wasserstoffatom oder eine Methylgruppe,
$R^2$ für eine Hydroxygruppe, eine $C_1$-$C_{10}$-Alkoxy- oder $C_1$-$C_{10}$-Acyloxygruppe,
$R^3$ für ein Wasserstoffatom, die Gruppierung $-(CH_2)_nCH_2Z$, wobei n 0, 1, 2, 3, 4 oder 5 ist, Z ein Wasserstoffatom, die Cyanogruppe oder den Rest $-OR^5$ mit $R^5$=H, $C_1$-$C_{10}$-Alkyl oder $C_1$-$C_{10}$-Acyl bedeuten, die Gruppierung $-(CH_2)_mC{\equiv}C-Y$, wobei m 0, 1 oder 2 und Y ein Wasserstoff-, Fluor-, Chlor- Brom-oder Jod-Atom, einen $C_1$-$C_{10}$-Hydroxyalkyl-, $C_1$-$C_{10}$-Alkoxyalkyl-, $C_1$-$C_{10}$-Acyloxyalkylrest bedeuten, die Gruppierung $-(CH_2)_p-CH{=}CH-(CH_2)_kCH_2R^6$, wobei p 0 oder 1 und k 0, 1 oder 2 und $R^6$ ein Wasserstoffatom, eine Hydroxygruppe, einen $C_1$-$C_4$-Alkoxy- oder $C_1$-$C_4$-Acyloxyrest bedeuten,
oder aber $R^2$ und $R^3$ gemeinsam für einen Rest der Formel

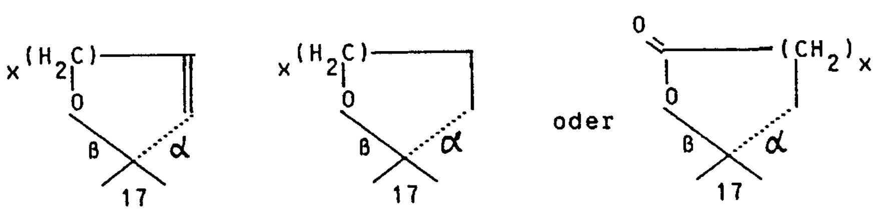

wobei x = 1 oder 2 ist

$R^4$ für ein Wasserstoffatom, eine Cyanogruppe, ein Chlor-, Fluor-, Brom-, Jodatom, für eine Trialkylsilyl-, Trialkylstannylgruppe, für einen geradkettigen oder verzweigten, gesättigten oder ungesättigten $C_1$-$C_8$-Alkyl-, -Acyl- oder Alkoxyalkylrest, für eine Aminogruppe

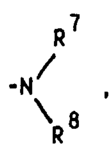

,

in welcher $R^7$ und $R^8$ unabhängig voneinander ein Wasserstoffatom oder eine $C_1$-$C_4$-Alkylgruppe bedeuten, oder für ein entsprechendes Aminoxid

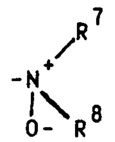,

oder für die Gruppierungen -$OR^9$ oder -$S(O)_iR^9$ mit i = 0, 1 oder 2, in welchen $R^9$ ein Wasserstoffatom, eine Methyl-, Ethyl-, Propyl-, Isopropyl-, Methoxyphenyl-, Allyl-oder eine 2-Dimethylaminoethylgruppe bedeuten, oder für einen Heteroarylrest der Formel Iα

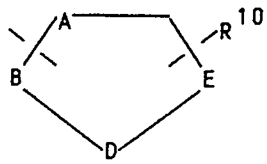

(Iα),

in welchem A ein Stickstoff-, Sauerstoff- oder Schwefelatom, -B-D-E- die Elementenfolge -C-C-C-, -N-C-C- oder -C-N-C- und $R^{10}$ ein Wasserstoffatom, eine Cyanogruppe, ein Chlor-, Fluor-, Brom-, Jodatom, eine Trialkylsilyl-, Trialkylstannylgruppe, einen geradkettigen oder verzweigten, gesättigten oder ungesättigten $C_1$-$C_8$-Alkyl-, -Acyl- oder Alkoxyalkylrest, für eine Aminogruppe

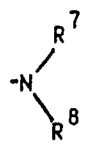,

in welcher $R^7$ und $R^8$ unabhängig voneinander ein Wasserstoffatom oder eine $C_1$-$C_4$-Alkylgruppe bedeuten, oder ein entsprechendes Aminoxid

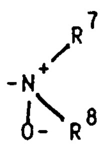,

oder die Gruppierung -$OR^9$ oder -$S(O)_iR^9$ mit i = 0, 1 oder 2, in welchen $R^9$ ein Wasserstoffatom, eine Methyl-, Ethyl-, Propyl-, Isopropyl-, Methoxyphenyl-, Allyl-oder eine 2-Dimethylaminoethylgruppe bedeuten,
symbolisieren,
oder für einen Heteroarylrest der Formel Iβ

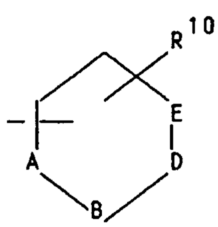

(Iβ),

in welchem A ein Stickstoffatom und -B-D-E- die Elementenfolge -C-C-C-, -N-C-C-, -C-N-C- oder -C-C-N- bedeuten und $R^{10}$ die bereits angegebene Bedeutung hat,
oder für einen Phenylrest der Formel Iγ

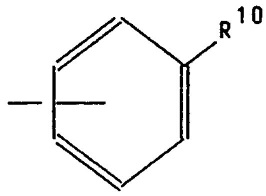

(Iγ),

worin $R^{10}$ die bereits angegebene Bedeutung hat,
stehen,
sowie deren pharmakologisch verträgliche Additionssalze mit Säure, dadurch gekennzeichnet, daß eine Verbindung der allgemeinen Formel II

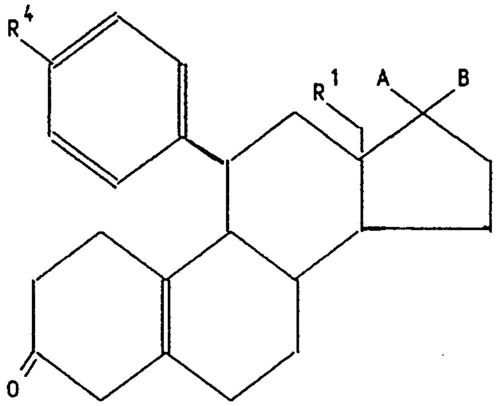

(II),

worin
$R^1$ und $R^4$ die in Formel I angegebene Bedeutung haben,
A für eine β-Hydroxygruppe oder den Rest $OR^2$ und
B für ein α-Wasserstoffatom, einen α-ständigen Rest $R^3$ oder
A und B gemeinsam für ein Wasserstoffatom stehen,
durch Erhitzen in Gegenwart von Säure in eine Verbindung der allgemeinen Formel Ia

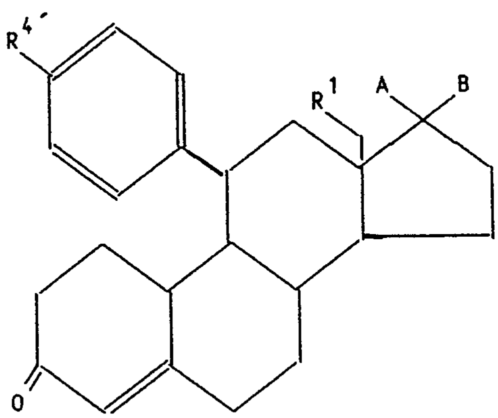

(Ia),

worin $R^1$, A und B die in Formel II angegebene Bedeutung haben und $R^{4'}$ dieselbe Bedeutung hat wie $R^4$ in Formel I, mit der Maßgabe, daß $R^4$ unter den genannten drastischen Reaktionsbedingungen stabil ist, überführt und anschließend entweder

a) in der Verbindung der allgemeinen Formel Ia, wenn darin A für eine β-Hydroxygruppe und B für ein α-Wasserstoffatom stehen, gewünschtenfalls die 17-Hydroxy- zur 17-Ketogruppe oxidiert und

b) die 3-Ketofunktion in ein Dithioketal überführt, wobei auch alle anderen, gegebenenfalls vorhandenen Ketogruppen ketalisiert werden oder aber zuerst b) und dann a) ausführt und danach

c) für den Fall, daß $R^{4'}$ in der 3-thioketalisierten Verbindung für eine Methoxy-oder eine Hydroxygruppe steht und $R^4$ in der letztlich gewünschten Verbindung der allgemeinen Formel I nicht für eine Methoxy- oder Hydroxygruppe stehen soll,

die Hydroxyverbindung, gegebenenfalls nach Spaltung der Methoxyverbindung, in eine entsprechende Perfluoralkylsulfonsäureverbindung, in welcher -alkyl- für einen $C_1$-$C_4$-Alkylrest steht, überführt und aus dieser entweder

direkt durch Umsetzung mit einer entsprechend substituierten Zinn(trialkyl)verbindung $R^{4''}$—$Sn(Alkyl)_3$ oder mit einer entsprechend substituierten Borverbindung $R^{4''}$—$BL_2$ (L=Hydroxy oder Alkyl), in welchen $R^{4''}$ mit $R^4$ der allgemeinen Formel I identisch ist oder einen tautomeren Vorläufer von $R^4$ darstellt und Alkyl einen $C_1$-$C_4$-Alkylrest bedeutet oder indirekt über eine in 4-Stellung des 11β-Phenylrestes mit einem Zinn(trialkyl)rest (alkyl = $C_1$-$C_4$) substituierte Verbindung, die durch Umsetzung der Perfluoralkylsulfonatverbindung mit $Sn_2alkyl_6$, erhalten wurde, und Weiterbehandlung der 11β-(4-Trialkylstannyl)-phenyl-Verbindung mit einer Verbindung $R^{4''}$-Y, worin $R^{4''}$ mit $R^4$ der allgemeinen Formel I identisch ist oder einen tautomeren Vorläufer von $R^4$ darstellt und Y eine Abgangsgruppe, vorzugsweise ein Halogenatom und insbesondere ein Bromatom bedeutet, in Gegenwart eines Übergangsmetall-Katalysators eine Verbindung der allgemeinen Formel III

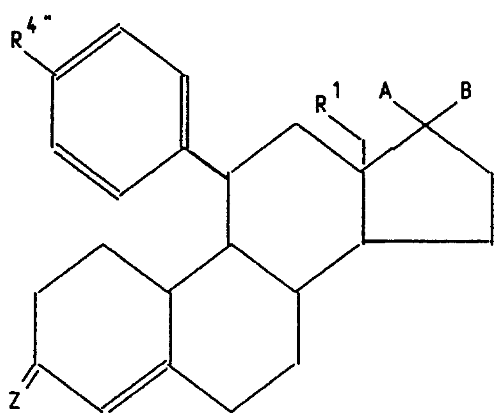

(III),

worin Z eine in Form eines Dithioketals geschützte Ketogruppe bedeutet, hergestellt und

d) dann, falls $R^2$ und $R^3$ in der letztlich gewünschten Verbindung der allgemeinen Formel I nicht für eine Hydroxygruppe bzw. ein Wasserstoffatom oder aber $R^2$ und $R^3$ gemeinsam nicht für ein Ketosauerstoffatom stehen sollen, am C-17-Atom des Steroidgerüstes die gewünschten Substituenten $R^2$ und $R^3$ nach an sich bekannten Methoden eingeführt

oder aber zuerst d) und dann c) ausgeführt wird,

Schutzgruppen abgespalten, gewünschtenfalls freie Hydroxygruppen alkyliert bzw. acyliert werden und gewünschtenfalls mit Hydroxylaminhydrochlorid die 3-Ketogruppe in eine 3-Hydroxyiminogruppierung >N~OH oder die 3-Ketogruppe in die Dihydroverbindung überführt sowie gegebenenfalls ein pharmazeutisch verträgliches Additionssalz mit einer Säure hergestellt wird.

**4.** Verfahren nach Anspruch 4, dadurch gekennzeichnet, daß auf eine Temperatur von 80-120°C erhitzt wird.

**5.** Verfahren nach Anspruch 3 oder 4, dadurch gekennzeichnet, daß in Gegenwart einer Mineralsäure erhitzt wird.

**6.** Verfahren nach Anspruch 3 oder 4, dadurch gekennzeichnet, daß in Gegenwart einer organischen Säure erhitzt wird.

**7.** Verfahren nach Anspruch 6, dadurch gekennzeichnet, daß in Gegenwart von p-Toluolsulfonsäure erhitzt wird.

**8.** Pharmazeutische Präparate, dadurch gekennzeichnet, daß sie mindestens eine Verbindung gemäß den Ansprüchen 1 oder 2 sowie einen pharmazeutisch verträglichen Träger enthalten.

**9.** Verwendung von Verbindungen gemäß den Ansprüchen 1 oder 2 zur Herstellung von Arzneimitteln.

## Claims

**1.** 11β-Aryl-4-oestrenes of the general formula I

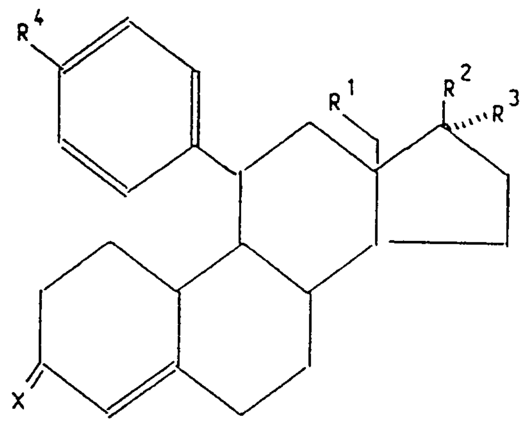

(I),

wherein

| | |
|---|---|
| X | is an oxygen atom, the hydroxyimino grouping >N~OH or two hydrogen atoms, |
| $R^1$ | is a hydrogen atom or a methyl group, |
| $R^2$ | is a hydroxy group or a $C_1$-$C_{10}$-alkoxy or $C_1$-$C_{10}$-acyloxy group, |
| $R^3$ | is a hydrogen atom; the grouping $-(CH_2)_nCH_2Z$ wherein n is 0, 1, 2, 3, 4 or 5, Z is a hydrogen atom, the cyano group or the radical $-OR^5$ wherein $R^5$ = H, $C_1$-$C_{10}$-alkyl or $C_1$-$C_{10}$-acyl; the grouping $-(CH_2)_mC\equiv C-Y$ wherein m is 0, 1 or 2 and Y is a hydrogen, fluorine, chlorine, bromine or iodine atom or a $C_1$-$C_{10}$-hydroxyalkyl, $C_1$-$C_{10}$-alkoxyalkyl or $C_1$-$C_{10}$-acyloxyalkyl radical; or the grouping $-(CH_2)_p-CH=CH-(CH_2)_kCH_2R^6$ wherein p is 0 or 1 and k is 0, 1 or 2 and $R^6$ is a hydrogen atom, a hydroxy group or a $C_1$-$C_4$-alkoxy or $C_1$-$C_4$-acyloxy radical; or |
| $R^2$ and $R^3$ | together form a radical of the formula |

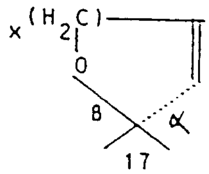

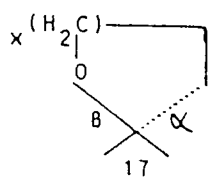

or

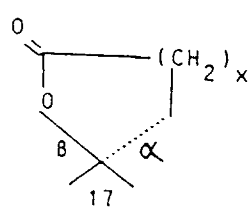

wherein x = 1 or 2,

$R^4$ is a hydrogen atom, a cyano group, a chlorine, fluorine, bromine or iodine atom, a trialkylsilyl or trialkylstannyl group, a straight-chain or branched, saturated or unsaturated $C_1$-$C_8$-alkyl, -acyl or -alkoxyalkyl radical, an amino group

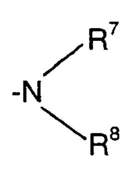

wherein $R^7$ and $R^8$ are each independently of the other a hydrogen atom or a $C_1$-$C_4$-alkyl group, or a corresponding amine oxide

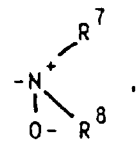

or the grouping $-OR^9$ or $-S(O)_iR^9$ wherein i = 0, 1 or 2 and $R^9$ is a hydrogen atom or a methyl, ethyl, propyl, isopropyl, methoxyphenyl, allyl or 2-dimethylaminoethyl group, or a heteroaryl radical of the formula Iα

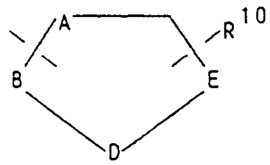

(Iα)

wherein A is a nitrogen, oxygen or sulphur atom, -B-D-E- is the element sequence -C-C-C-, -N-C-C- or -C-N-C- and $R^{10}$ is a hydrogen atom, a cyano group, a chlorine, fluorine, bromine or iodine atom, a trialkylsilyl or trialkylstannyl group, a straight-chain or branched, saturated or unsaturated $C_1$-$C_8$-alkyl, -acyl or -alkoxyalkyl radical, an amino group

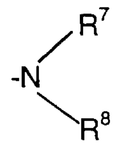

wherein $R^7$ and $R^8$ are each independently of the other a hydrogen atom or a $C_1$-$C_4$-alkyl group, or a corresponding amine oxide

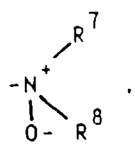

or the grouping $-OR^9$ or $-S(O)_iR^9$ wherein i = 0, 1 or 2 and $R^9$ is a hydrogen atom or a methyl, ethyl, propyl, isopropyl, methoxyphenyl, allyl or 2-dimethylaminoethyl group, or a heteroaryl radical of the formula Iβ

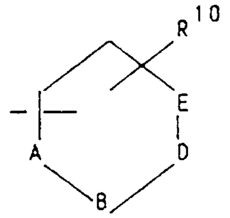

(Iβ)

wherein A is a nitrogen atom and -B-D-E- is the element sequence - C-C-C-, -N-C-C-, -C-N-C- or - C-C-N- and $R^{10}$ has the meaning given above, or a phenyl radical of the formula Iγ

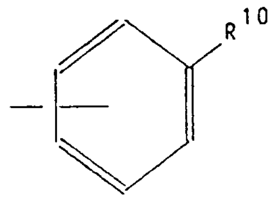

(Iγ)

wherein $R^{10}$ has the meaning given above,

and their pharmacologically acceptable addition salts with acids.

**2.** 11β-[4-(Dimethylamino)phenyl]-17β-hydroxy-17α-(1-propynyl)-4-oestren-3-one;
11β-(4-acetylphenyl)-17β-hydroxy-4-oestren-3-one;
11β-(4-acetylphenyl)-17β-hydroxy-17α-(1-propynyl)-4-oestren-3-one;
11β-(4-acetylphenyl)-17β-hydroxy-17α-(3-hydroxy-1Z-propenyl)-4-oestren-3-one;
11β-4-(acetylphenyl)-17β-hydroxy-17α-(3-hydroxy-1Z-propenyl)-4-oestren-3-one;
11β-[4-(dimethylamino)phenyl]-17β-hydroxy-17α-(3-hydroxy-1Z-propenyl)-4-oestren-3-one;
11β-[4-(3-furyl)phenyl]-17β-hydroxy-17α-(1-propynyl)-4-oestren-3-one;
11β-[4-(3-furyl)phenyl]-17β-hydroxy-17α-(3-hydroxy-1Z-propenyl)-4-oestren-3-one;
11β-[4-(5-pyrimidinyl)phenyl]-17β-hydroxy-17α-(1-propynyl)-4-oestren-3-one;
11β-[4-(5-pyrimidinyl)phenyl]-17β-hydroxy-17α-(3-hydroxy-1Z-propenyl)-4-oestren-3-one;
11β-[4-(3-pyridyl)phenyl]-17β-hydroxy-17α-(1-propynyl)-4-oestren-3-one;
11β-[4-(3-pyridyl)phenyl]-17β-hydroxy-17α-(3-hydroxy-1Z-propenyl)-4-oestren-3-one;
11β-[4-(4-cyanophenyl)phenyl]-17β-hydroxy-17α-(1-propynyl)-4-oestren-3-one;
11β-[4-(4-cyanophenyl)phenyl]-17β-hydroxy-17α-(3-hydroxy-1Z-propenyl)-4-oestren-3-one;
11β-(4-vinylphenyl)-17β-hydroxy-17α-(3-hydroxy-1Z-propenyl)-4-oestren-3-one;
11β-(4-vinylphenyl)-17β-hydroxy-17α-(1-propynyl)-4-oestren-3-one;
11β-[4-(1-hydroxyethyl)phenyl]-17β-hydroxy-17α-(3-hydroxy-1Z-propenyl)-4-oestren-3-one;
11β-[4-(dimethylamino)phenyl]-17β-hydroxy-17α-methoxymethyl-4-oestren-3-one;
11β-[4-(dimethylamino)phenyl]-17β-hydroxy-17α-cyanomethyl-4-oestren-3-one;
(11β,17β)-4',5'-dihydro-11-[4-(dimethylamino)phenyl]spiro[oestr-4-ene-17,2'(3'H)-furan]-3-one;
(11β,17β)-3',4'-dihydro-11-[4-(dimethylamino)phenyl]spiro[oestr-4-ene-17,2'(3'H)-furan]-3,5'-dione;
(11β,17β)-11-[4-(dimethylamino)phenyl]spiro[oestr-4-ene-17,2'(5'H)-furan]-3-one;
11β-[4-(dimethylamino)phenyl]-17α-(1-propynyl)-4-oestren-17β-ol;
17β-hydroxy-3-oxo-11β-[4-(3-pyridinyl)phenyl]-4-oestrene-17α-acetonitrile;
(*E*)-17β-hydroxy-3-(hydroxyimino)-11β-[4-(3-pyridinyl)phenyl]-4-oestrene-17α-acetonitrile;
(*Z*)-17β-hydroxy-3-(hydroxyimino)-11β-[4-(3-pyridinyl)phenyl]-4-oestrene-17α-acetonitrile;
17β-hydroxy-17α-(2-propenyl)-11β-[4-(3-pyridinyl)phenyl]-4-oestren-3-one;
17β-hydroxy-17α-(methoxymethyl)-11β-[4-(3-pyridinyl)phenyl]-4-oestren-3-one;
11β-(4-ethylphenyl)-17β-hydroxy-17α-(1-propynyl)-4-oestren-3-one;
(*Z*)-11β-(4-ethylphenyl)-17β-hydroxy-17α-(3-hydroxy-1-propenyl)-4-oestren-3-one;
(*Z*)-11β-[4-(2-furanyl)phenyl]-17β-hydroxy-17α-(3-hydroxy-1-propenyl)-4-oestren-3-one;
11β-(4-ethenylphenyl)-17β-hydroxy-17α-methyl-4-oestren-3-one;
(*Z*)-17β-hydroxy-17α-(3-hydroxy-1-propenyl)-11β-(4-methylphenyl)-4-oestren-3-one;
(11β,17β)-11-[4-(5-pyrimidinyl)phenyl]spiro[oestr-4-ene-17,2'(3'H)-furan]-3-one.

**3.** A process for the preparation of 11β-aryl-4-oestrenes of the general formula I

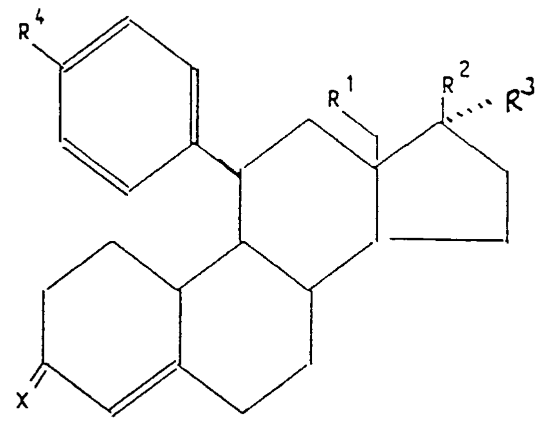

(I),

wherein

X is an oxygen atom, the hydroxyimino grouping >N~OH or two hydrogen atoms,

$R^1$ is a hydrogen atom or a methyl group,

$R^2$ is a hydroxy group or a $C_1$-$C_{10}$-alkoxy or $C_1$-$C_{10}$-acyloxy group,

$R^3$ is a hydrogen atom; the grouping $-(CH_2)_nCH_2Z$ wherein n is 0, 1, 2, 3, 4 or 5, Z is a hydrogen atom, the cyano group or the radical $-OR^5$ wherein $R^5$ = H, $C_1$-$C_{10}$-alkyl or $C_1$-$C_{10}$-acyl; the grouping $-(CH_2)_mC{\equiv}C\text{-}Y$ wherein m is 0, 1 or 2 and Y is a hydrogen, fluorine, chlorine, bromine or iodine atom or a $C_1$-$C_{10}$-hydroxyalkyl, $C_1$-$C_{10}$-alkoxyalkyl or $C_1$-$C_{10}$-acyloxyalkyl radical; or the grouping $-(CH_2)_p\text{-}CH{=}CH\text{-}(CH_2)_kCH_2R^6$ wherein p is 0 or 1 and k is 0, 1 or 2 and $R^6$ is a hydrogen atom, a hydroxy group or a $C_1$-$C_4$-alkoxy or $C_1$-$C_4$-acyloxy radical; or

$R^2$ and $R^3$ together form a radical of the formula

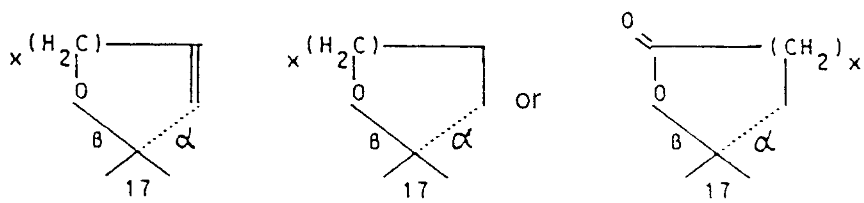

wherein x = 1 or 2,

$R^4$ is a hydrogen atom, a cyano group, a chlorine, fluorine, bromine or iodine atom, a trialkylsilyl or trialkylstannyl group, a straight-chain or branched, saturated or unsaturated $C_1$-$C_8$-alkyl, -acyl or -alkoxyalkyl radical, an amino group

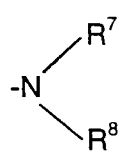

wherein $R^7$ and $R^8$ are each independently of the other a hydrogen atom or a $C_1$-$C_4$-alkyl group, or a corresponding amine oxide

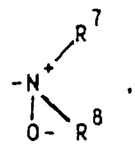

or the grouping $-OR^9$ or $-S(O)_iR^9$ wherein i = 0, 1 or 2 and $R^9$ is a hydrogen atom or a methyl, ethyl, propyl, isopropyl, methoxyphenyl, allyl or 2-dimethylaminoethyl group, or a heteroaryl radical of the formula Iα

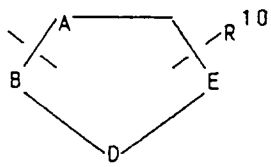

(Iα)

wherein A is a nitrogen, oxygen or sulphur atom, -B-D-E- is the element sequence -C-C-C-, -N-C-C- or -C-N-C- and $R^{1°}$ is a hydrogen atom, a cyano group, a chlorine, fluorine, bromine or iodine atom, a trialkylsilyl or trialkylstannyl group, a straight-chain or branched, saturated or unsaturated $C_1$-$C_8$-alkyl, -acyl or -alkoxyalkyl radical, an amino group

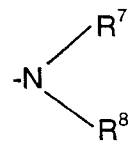

wherein $R^7$ and $R^8$ are each independently of the other a hydrogen atom or a $C_1$-$C_4$-alkyl group, or a corresponding amine oxide

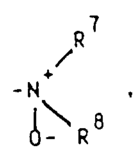

or the grouping $-OR^9$ or $-S(O)_iR^9$ wherein i = 0, 1 or 2 and $R^9$ is a hydrogen atom or a methyl, ethyl, propyl, isopropyl, methoxyphenyl, allyl or 2-dimethylaminoethyl group,
or a heteroaryl radical of the formula Iβ

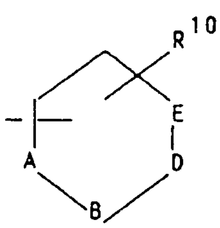

(Iβ)

wherein A is a nitrogen atom and -B-D-E- is the element sequence - C-C-C-, -N-C-C-, -C-N-C- or -C-C-N- and $R^{10}$ has the meaning given above, or a phenyl radical of the formula Iγ

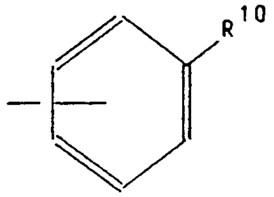

(Iγ)

wherein $R^{10}$ has the meaning given above,

and their pharmacologically acceptable addition salts with acids, characterised in that a compound of the general formula II

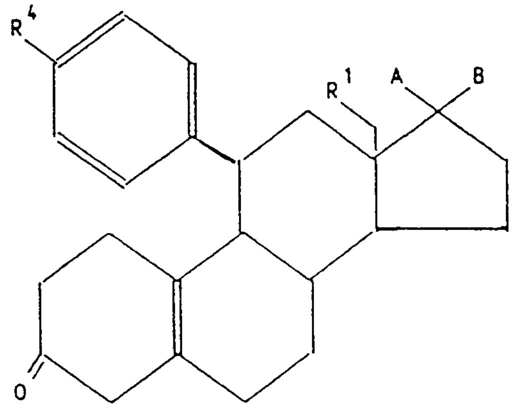

(II),

wherein
$R^1$ and $R^4$ have the meanings given in formula I,
A is a β-hydroxy group or the radical $OR^2$ and
B is an α-hydrogen atom or a radical $R^3$ in the α-configuration, or
A and B together are a hydrogen atom,
is converted by heating in the presence of an acid into a compound of the general formula Ia

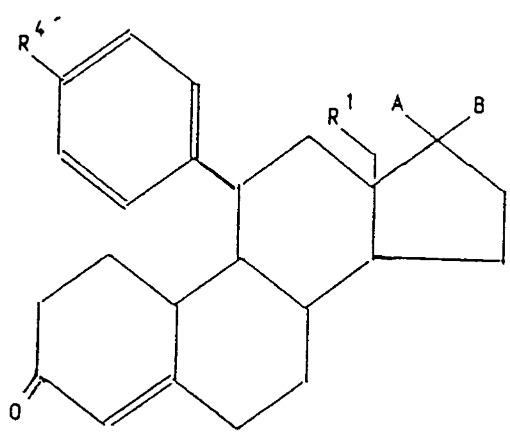

(Ia)

wherein $R^1$, A and B have the meanings given in formula II and $R^{4'}$ has the same meaning as $R^4$ in formula I, with the proviso that $R^4$ is stable under the drastic reaction conditions mentioned, and then either

a) in the compound of the general formula Ia, when A in that compound is a β-hydroxy group and B is an α-hydrogen atom, if desired the 17-hydroxy group is oxidised to a 17-keto group and

b) the 3-keto function is converted into a dithioketal, all other keto groups which may be present also being ketalised, or

first b) and then a) are carried out and then

c) in the case where $R^{4'}$ in the 3-thioketalised compound is a methoxy or hydroxy group and $R^4$ in the ultimately desired compound of the general formula I is not to be a methoxy or hydroxy group,

the hydroxy compound, optionally after cleavage of the methoxy compound, is converted into a corresponding perfluoroalkylsulphonic acid compound in which alkyl is a $C_1$-$C_4$-alkyl radical, and there is prepared therefrom either directly by reaction with a correspondingly substituted tin(trialkyl) compound $R^{4''}$—$Sn(alkyl)_3$ or with a correspondingly substituted boron compound $R^{4''}$—$BL_2$ (L = hydroxy or alkyl) wherein $R^{4''}$ is identical to $R^4$ in the general formula I or is a tautomeric precursor of $R^4$ and alkyl is a $C_1$-$C_4$-alkyl radical or indirectly by way of a compound substituted in the 4-position of the 11β-phenyl radical by a tin(trialkyl) radical (alkyl = $C_1$-$C_4$), which compound has been obtained by reaction of the perfluoroalkylsulphonate compound with $Sn_2alkyl_6$, and by further treatment of the 11β-(4-trialkylstannyl)-phenyl compound with a compound $R^{4''}$-Y wherein $R^{4''}$ is identical to $R^4$ in the general formula I or is a tautomeric precursor of $R^4$ and Y is a leaving group, preferably a halogen atom and especially a bromine atom, in the presence of a transition metal catalyst, a compound of the general formula III

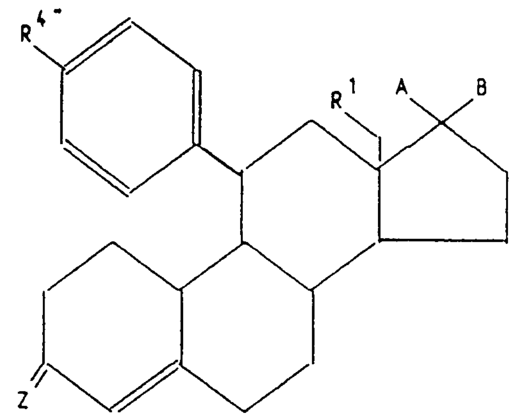

(III)

wherein Z is a keto group protected in the form of a dithioketal, and

d) then, if $R^2$ and $R^3$ in the ultimately desired compound of the general formula I are not to be a hydroxy group and a hydrogen atom, respectively, or alternatively $R^2$ and $R^3$ together are not to be a keto oxygen atom, the desired substituents $R^2$ and $R^3$ are introduced at the C17 atom of the steroid structure in accordance with methods known *per se*,

or first d) and then c) are carried out,
protecting groups are removed, if desired free hydroxy groups are alkylated or acylated and, if desired, using hydroxylamine hydrochloride the 3-keto group is converted into a 3-hydroxyimino grouping >N~OH or the 3-keto group is converted into the dihydro compound and optionally a pharmaceutically acceptable addition salt with an acid is prepared.

**4.** Process according to claim 4, characterised in that the heating is at a temperature of 80-120°C.

**5.** Process according to claim 3 or 4, characterised in that the heating is carried out in the presence of a mineral acid.

**6.** Process according to claim 3 or 4, characterised in that the heating is carried out in the presence of an organic acid.

**7.** Process according to claim 6, characterised in that the heating is carried out in the presence of p-toluenesulphonic acid.

**8.** Pharmaceutical preparations, characterised in that they contain at least one compound according to claim 1 or 2 and a pharmaceutically acceptable carrier.

**9.** Use of compounds according to claim 1 or 2 for the manufacture of medicaments.

**Revendications**

**1.** 11β-aryl-4-estrènes de formule générale I

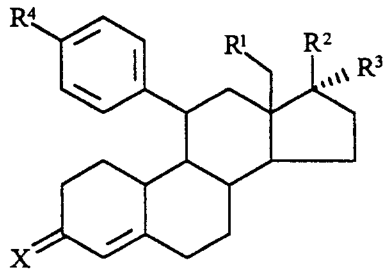

(I),

dans laquelle

X représente un atome d'oxygène, le groupement hydroxyimino >N~OH ou deux atomes d'hydrogène,

$R^1$ représente un atome d'hydrogène ou un groupe méthyle,

$R^2$ représente un groupe hydroxy, alcoxy en $C_1$-$C_{10}$ ou acyloxy en $C_1$-$C_{10}$,

$R^3$ représente un atome d'hydrogène, le groupement $-(CH_2)_nCH_2Z$, dans lequel n est égal à 0, 1, 2, 3, 4 ou 5, Z est un atome d'hydrogène, le groupe cyano ou le reste $-OR^5$ avec $R^5$ = H, alkyle en $C_1$-$C_{10}$ ou acyle en $C_1$-$C_{10}$, le groupement $-(CH_2)_mC{\equiv}C-Y$ dans lequel m est égal à 0, 1 ou 2 et Y est un atome d'hydrogène, de fluor, de chlore, de brome ou d'iode ou un reste hydroxyalkyle en $C_1$-$C_{10}$, alcoxyalkyle en $C_1$-$C_{10}$ ou acyloxyalkyle en $C_1$-$C_{10}$, le groupement $-(CH_2)_p-CH=CH-(CH_2)_kCH_2R^6$ dans lequel p est 0 ou 1 et k est 0, 1 ou 2 et $R^6$ est un atome d'hydrogène, un groupe hydroxy ou un reste alcoxy en $C_1$-$C_4$ ou acyloxy en $C_1$-$C_4$,

ou bien $R^2$ et $R^3$ forment ensemble un reste de formule

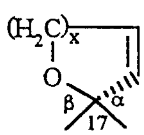

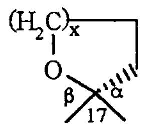

ou

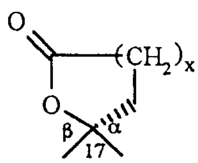

où x= 1 ou 2,

$R^4$ représente un atome d'hydrogène, un groupe cyano, un atome de chlore, de fluor, de brome ou d'iode, un groupe trialkylsilyle, un groupe trialkylstannyle, un reste alkyle, acyle ou alcoxyalkyle en $C_1$-$C_8$ linéaire ou ramifié, saturé ou insaturé, un groupe amino

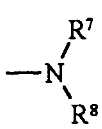

dans lequel $R^7$ et $R^8$ représentent indépendamment l'un de l'autre un atome d'hydrogène ou un groupe alkyle en $C_1$-$C_4$, ou un oxyde d'amine correspondant

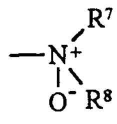 ,

ou les groupements $-OR^9$ ou $-S(O)_iR^9$ avec i = 0, 1 ou 2, dans lesquels $R^9$ représente un atome d'hydrogène ou un groupe méthyle, éthyle, propyle, isopropyle, méthoxyphényle, allyle ou 2-diméthylaminoéthyle, ou un reste hétéroaryle de formule Iα

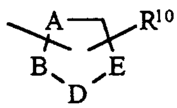 (Iα),

dans laquelle A représente un atome d'azote, d'oxygène ou de soufre, -B-D-E- représente la séquence d'éléments -C-C-C-, -N-C-C- ou -C-N-C- et $R^{10}$ est un atome d'hydrogène, un groupe cyano, un atome de chlore, de brome, de fluor ou d'iode, un groupe trialkylsilyle, un groupe trialkylstannyle, un reste alkyle, acyle ou alcoxyalkyle en $C_1$-$C_8$ linéaire ou ramifié, saturé ou insaturé, un groupe amino

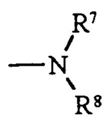

dans lequel $R^7$ et $R^8$ représentent indépendamment l'un de l'autre un atome d'hydrogène ou un groupe alkyle en $C_1$-$C_4$, ou un oxyde d'amine correspondant

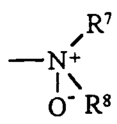 ,

ou les groupements $-OR^9$ ou $-S(O)_iR^9$ avec i = 0, 1 ou 2, dans lesquels $R^9$ représente un atome d'hydrogène ou un groupe méthyle, éthyle, propyle, isopropyle, méthoxyphényle, allyle ou 2-diméthylaminoéthyle, ou un reste hétéroaryle de formule 1β

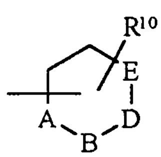 (Iβ),

dans laquelle A est un atome d'azote et -B-D-E- représente la séquence d'éléments -C-C-C-, -N-C-C-, -C-N-C- ou -C-C-N- et $R^{10}$ a la signification déjà indiquée,
ou un reste phényle de formule Iγ

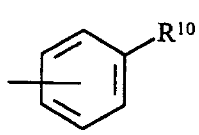 (Iγ),

dans laquelle $R^{10}$ a la signification déjà indiquée,

et leurs sels d'addition d'acides pharmaceutiquement acceptables.

**2.** La 11β-[4-(diméthylamino)phényl]-17β-hydroxy-17α-(1-propynyl)-4-estrène-3-one;
la 11β-(4-acétylphényl)-17β-hydroxy-4-estrène-3-one;
la 11β-(4-acétylphényl)-17β-hydroxy-17α-(1-propynyl)-4-estrène-3-one;
la 11β-(4-acétylphényl)-17β-hydroxy-17α-(3-hydroxy-1Z-propényl)-4-estrène-3-one;
la 11β-(4-acétylphényl)-17β-hydroxy-17α-(3-hydroxy1Z-propényl)-4-estrène-3-one;
la 11β-[4-(diméthylamino)phényl]-17β-hydroxy-17α-(3-hydroxy-1Z-propényl)-4-estrène-3-one;
la 11β-[4-(3-furyl)phényl]-17β-hydroxy-17α-(1-propynyl)-4-estrène-3-one;
la 11β-[4-(3-furyl)phényl]-17β-hydroxy-17α-(3-hydroxy-1Z-propényl)-4-estrène-3-one;
la 11β-[4-(5-pyrimidinyl)phényl]-17β-hydroxy-17α-(1-propynyl)-4-estrène-3-one;
la 11β-[4-(5-pyrimidinyl)phényl]-17β-hydroxy-17α-(3-hydroxy-1Z-propényl)-4-estrène-3-one;
la 11β-[4-(3-pyridyl)phényl]-17β-hydroxy-17α-(1-propynyl)-4-estrène-3-one; la 11β-[4-(3-pyridyl)phényl]-17β-hydroxy-17α-(3-hydroxy-1Z-propényl)-4-estrène-3-one;
la 11β-[4-(4-cyanophényl)phényl]-17β-hydroxy-17α-(1-propynyl)-4-estrène-3-one;
la 11β-[4-(4-cyanophényl)phényl]-17β-hydroxy-17α-(3-hydroxy-1Z-propényl)-4-estrène-3-one;
la 11β-(4-vinylphényl)-17β-hydroxy-17α-(3-hydroxy-1Z-propényl)-4-estrène-3-one;
la 11β-(4-vinylphényl)-17β-hydroxy-17α-(1-propynyl)-4-estrène-3-one;
la 11β-[4-(1-hydroxyéthyl)phényl]-17β-hydroxy-17α-(3-hydroxy-1Z-propényl)-4-estrène-3-one;
la 11β-[4-(diméthylamino)phényl]-17β-hydroxy-17α-méthoxyméthyl-4-estrène-3-one;
la 11β-[4-(diméthylamino)phényl]-17β-hydroxy-17α-cyanométhyl-4-estrène-3-one;
la (11β,17β)-4',5'-dihydro-11-[4-(diméthylamino)phényl]spiro[estr-4-ène-17,2'(3'H)furane]-3-one;
la (11β,17β)-3',4'-dihydro-11-[4-(diméthylamino)phényl]spiro[estr-4-ène-17,2'(5'H)furane]-3,5'-dione;
la (11β,17β)-11-[4-(diméthylamino)phényl]spiro[estr-4-ène-17,2'(5'H)furane]-3-one;
le 11β-[4-(diméthylamino)phényl]-17α-(1-propynyl)-4-estrène-17β-ol;
le 17β-hydroxy-3-oxo-11β-[4-(3-pyridinyl)phényl]-4-estrène-17α-acétonitrile;
le (*E*)-17β-hydroxy-3-(hydroxyimino)-11β-[4-(3-pyridinyl)phényl]-4-estrène-17α-acétonitrile;
le (*Z*)-17β-hydroxy-3-(hydroxyimino)-11β-[4-(3-pyridinyl)phényl]-4-estrène-17α-acétonitrile;
la 17β-hydroxy-17α-(2-propényl)-11β-[4-(3-pyridinyl)phényl]-4-estrène-3-one;
la 17β-hydroxy-17α-(méthoxyméthyl)-11β-[4-(3-pyridinyl)phényl]-4-estrène-3-one;
la 11β-(4-éthylphényl)-17β-hydroxy-17α-(1-propynyl)-4-estrène-3-one;
la (*Z*)-11β-(4-éthylphényl)-17β-hydroxy-17α-(3-hydroxy-1-propényl)-4-estrène-3-one;
la (*Z*)-11β-[4-(2-furanyl)phényl]-17β-hydroxy-17α-(3-hydroxy-1-propényl)-4-estrène-3-one;
la 11β-(4-éthénylphényl)-17β-hydroxy-17α-méthyl-4-estrène-3-one;
la (*Z*)-17β-hydroxy-17α-(3-hydroxy-1-propényl)-11β-(4-méthylphényl)-4-estrène-3-one;
la (11β,17β)-11-[4-(5-pyrimidinyl)phényl]spiro[estr-4-ène-17,2'(3'H)furane]-3-one.

**3.** Procédé de préparation de 11β-aryl-4-estrènes de formule générale I

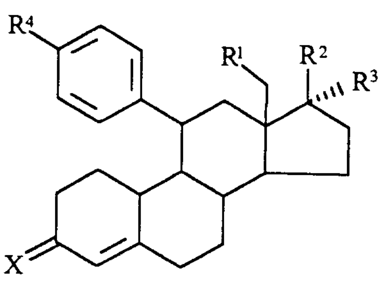

(I),

dans laquelle

X représente un atome d'oxygène, le groupement hydroxyimino >N~OH ou deux atomes d'hydrogène,
$R^1$ représente un atome d'hydrogène ou un groupe méthyle,
$R^2$ représente un groupe hydroxy, alcoxy en $C_1$-$C_{10}$ ou acyloxy en $C_1$-$C_{10}$,
$R^3$ représente un atome d'hydrogène, le groupement $-(CH_2)_nCH_2Z$, dans lequel n est égal à 0, 1, 2, 3, 4 ou 5, Z est un atome d'hydrogène, le groupe cyano ou le reste $-OR^5$ avec $R^5$ = H, alkyle en $C_1$-$C_{10}$ ou acyle en

$C_1$-$C_{10}$, le groupement $-(CH_2)_mC{\equiv}C-Y$ dans lequel m est égal à 0, 1 ou 2 et Y est un atome d'hydrogène, de fluor, de chlore, de brome ou d'iode ou un reste hydroxyalkyle en $C_1$-$C_{10}$, alcoxyalkyle en $C_1$-$C_{10}$ ou acyloxyalkyle en $C_1$-$C_{10}$, le groupement $-(CH_2)_p-CH{=}CH-(CH_2)_kCH_2R^6$ dans lequel p est 0 ou 1 et k est 0, 1 ou 2 et $R^6$ est un atome d'hydrogène, un groupe hydroxy ou un reste alcoxy en $C_1$-$C_4$ ou acyloxy en $C_1$-$C_4$,

ou bien $R^2$ et $R^3$ forment ensemble un reste de formule

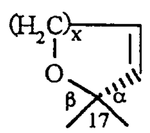
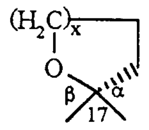
ou
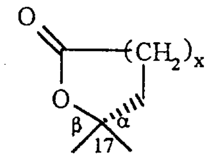

où x = 1 ou 2,

$R^4$ représente un atome d'hydrogène, un groupe cyano, un atome de chlore, de fluor, de brome ou d'iode, un groupe trialkylsilyle, un groupe trialkylstannyle, un reste alkyle, acyle ou alcoxyalkyle en $C_1$-$C_8$ linéaire ou ramifié, saturé ou insaturé, un groupe amino

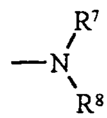

dans lequel $R^7$ et $R^8$ représentent indépendamment l'un de l'autre un atome d'hydrogène ou un groupe alkyle en $C_1$-$C_4$, ou un oxyde d'amine correspondant

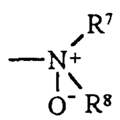
,

ou les groupements $-OR^9$ ou $-S(O)_iR^9$ avec i = 0, 1 ou 2, dans lesquels $R^9$ représente un atome d'hydrogène ou un groupe méthyle, éthyle, propyle, isopropyle, méthoxyphényle, allyle ou 2-diméthylaminoéthyle, ou un reste hétéroaryle de formule Iα

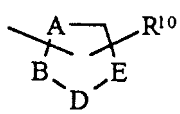
(Iα),

dans laquelle A représente un atome d'azote, d'oxygène ou de soufre, -B-D-E- représente la séquence d'éléments -C-C-C-, -N-C-C- ou -C-N-C- et $R^{10}$ est un atome d'hydrogène, un groupe cyano, un atome de chlore, de brome, de fluor ou d'iode, un groupe trialkylsilyle, un groupe trialkylstannyle, un reste alkyle, acyle ou alcoxyalkyle en $C_1$-$C_8$ linéaire ou ramifié, saturé ou insaturé, un groupe amino

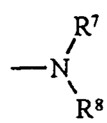

dans lequel $R^7$ et $R^8$ représentent indépendamment l'un de l'autre un atome d'hydrogène ou un groupe alkyle en $C_1$-$C_4$, ou un oxyde d'amine correspondant

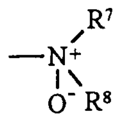 ,

ou les groupements $-OR^9$ ou $-S(O)_iR^9$ avec i = 0, 1 ou 2, dans lesquels $R^9$ représente un atome d'hydrogène ou un groupe méthyle, éthyle, propyle, isopropyle, méthoxyphényle, allyle ou 2-diméthylaminoéthyle, ou un reste hétéroaryle de formule 1β

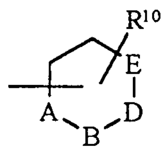 (Iβ),

dans laquelle A est un atome d'azote et -B-D-E- représente la séquence d'éléments -C-C-C-, -N-C-C-, -C-N-C- ou -C-C-N- et $R^{10}$ a la signification déjà indiquée,
ou un reste phényle de formule Iγ

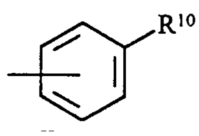 (Iγ),

dans laquelle $R^{10}$ a la signification déjà indiquée,

et de leurs sels d'addition d'acides pharmaceutiquement acceptables, caractérisé en ce que l'on transforme un composé de formule générale II

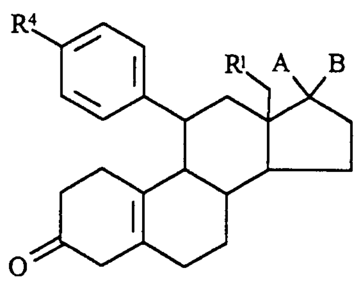 (II),

dans laquelle
$R^1$ et $R^4$ ont la signification indiquée pour la formule I,
A est un groupe β-hydroxy ou le reste $OR^2$, et
B est un atome d'hydrogène en α, un reste $R^3$ en α, ou
A et B représentent ensemble un atome d'hydrogène,
par chauffage en présence d'un acide, en un composé de formule générale la

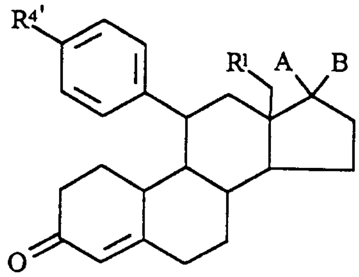

(Ia),

dans laquelle $R^1$, A et B ont la signification donnée pour la formule II et $R^{4'}$ a la même signification que $R^4$ dans la formule I, à condition que $R^4$ soit stable dans les conditions réactionnelles sévères indiquées, puis

a) dans le composé de formule générale (Ia) dans lequel A est un groupe hydroxy en β et B un atome d'hydrogène en α, on oxyde si désiré le groupe 17-hydroxy en groupe 17-céto, et
b) on transforme la fonction 3-céto en un dithiocétal, tous les autres groupes céto éventuellement présents étant aussi cétalisés, ou bien
on effectue d'abord l'étape b) puis l'étape a), puis
c) dans le cas où $R^{4'}$ dans le composé 3-thiocétalisé est un groupe méthoxy ou un groupe hydroxy et où $R^4$ ne doit pas être un groupe méthoxy ou hydroxy dans le composé final de formule générale I désiré,
on transforme le composé hydroxy, éventuellement après avoir dissocié le groupe méthoxy, en le composé d'acide perfluoroalkylsulfonique correspondant dans lequel la partie alkyle est un reste alkyle en $C_1$-$C_4$, et, à partir de celui-ci, on prépare, en présence d'un catalyseur à base d'un métal de transition, un composé de formule générale III

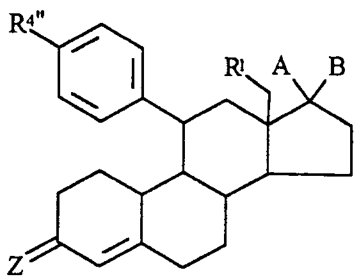

(III),

dans laquelle Z est un groupe céto protégé sous forme d'un dithiocétal,
soit directement par réaction avec un composé de trialkylétain substitué de façon correspondante $R^{4''}$-Sn(alkyl)$_3$ ou avec un composé de bore substitué de façon correspondante $R^{4''}$-$BL_2$ (L = hydroxy ou alkyle) dans lesquels $R^{4''}$ est identique au $R^4$ de la formule générale I ou représente un précurseur tautomère de $R^4$, et alkyle représente un reste alkyle en $C_1$-$C_4$, soit indirectement en passant par un composé substitué par un reste trialkylétain (alkyl = alkyle en $C_1$-$C_4$) en position 4 du reste phényle en 11β que l'on a obtenu par réaction du composé perfluoralkylsulfonate avec $Sn_2$alkyl$_6$, et en traitant ensuite le composé 11β-(4-trialkylstannyl)phényle avec un composé $R^{4''}$-Y dans lequel $R^{4''}$ est identique au $R^4$ de la formule générale I ou représente un précurseur tautomère de $R^4$ et Y est un groupe partant, de préférence un atome d'halogène et en particulier un atome de brome, et
d) ensuite, dans le cas où $R^2$ et $R^3$ dans le composé final de formule générale I désiré ne doivent pas représenter respectivement un groupe hydroxy et un atome d'hydrogène, ou bien dans le cas où $R^2$ et $R^3$ ne doivent pas représenter ensemble un atome d'oxygène d'un groupe céto, on introduit par des procédés connus en soi les substituants $R^2$ et $R^3$ désirés sur l'atome de carbone 17 du squelette du stéroïde, ou bien
on effectue d'abord l'étape d) puis l'étape c),
on sépare les groupes protecteurs, si désiré on alkyle ou on acyle les groupes hydroxy libres, et si désiré on transforme avec du chlorhydrate d'hydroxylamine le groupe 3-céto en un groupe 3-hydroxyimino >N~OH, ou on transforme le groupe 3-céto en le composé dihydro, et on prépare éventuellement un sel d'addition avec un acide pharmaceutiquement acceptable.

4. Procédé selon la revendication 4, caractérisé en ce que l'on chauffe à une température de 80-120°C.

5. Procédé selon la revendication 3 ou 4, caractérisé en ce que l'on chauffe en présence d'un acide minéral.

6. Procédé selon la revendication 3 ou 4, caractérisé en ce que l'on chauffe en présence d'un acide organique.

7. Procédé selon la revendication 6, caractérisé en ce que l'on chauffe en présence de l'acide p-toluènesulfonique.

8. Compositions pharmaceutiques, caractérisées en ce qu'elles contiennent au moins un composé selon les revendications 1 ou 2, et un support pharmaceutiquement acceptable.

9. Utilisation des composés selon les revendications 1 ou 2 pour la préparation de médicaments.